# EUROPEAN PATENT APPLICATION

(11) **EP 2 218 778 A1**
(43) Date of publication of application: **18.08.2010**
(21) Application number: 09002141.1
(22) Date of filing: 16.02.2009
(51) Int. Cl.: C12N 5/076

(54) **Conversion of unipotent germline stem cells into pluripotent stem cells without exogenous factors**

(71) Applicant: Max-Planck-Gesellschaft zur Förderung der Wissenschaften e.V., 80539 München (DE)
(72) Inventor: Ko, Kinarm, 48161 Münster (DE); Schöler, Hans Robert, 48151 Münster (DE)
(74) Representative: Vossius & Partner

(57) **Abstract**

The present invention relates to a method of generating pluripotent stem cells comprising the steps: (a) plating germline stem cells (GSCs) at a density of 20,000 cells or less per 1.9 cm²; and (b) culturing the germline stem cells for at least 11 days without subculturing while maintaining said germline stem cells in an undifferentiated state, wherein during or after step (b) the presence of markers characteristic of pluripotent stem cells is indicative of the generation of pluripotent stem cells. Also, the invention relates to a pluripotent stem cell obtainable by the method of the invention and a cell obtainable by differentiation of said pluripotent stem cell. Further, the invention relates to a method of generating a transgenic non-human animal and a transgenic non-human animal generated by said method. Finally, the invention envisions a composition comprising a pluripotent stem cell of the invention.

SEQUENCE LISTING

<110>  Max-Planck-Gesellschaft zur Förderung der Wissenschaften
       e.V.

<120>  Conversion of unipotent germline stem cells into pluripotent stem
       cells without exogenous factors

<130>  P1977 EP

<160>  62

<170>  PatentIn version 3.3

<210>  1
<211>  24
<212>  DNA
<213>  artificial sequence

<220>
<223>  /note="Description of artificial sequence: Oct3/4 forward primer"

<400>  1
ctgagggcca ggcaggagca cgag                                              24


<210>  2
<211>  24
<212>  DNA
<213>  artificial sequence

<220>
<223>  /note="Description of artificial sequence: Oct3/4 reverse primer"

<400>  2
ctgtagggag ggcttcgggc actt                                              24


<210>  3
<211>  22
<212>  DNA
<213>  artificial sequence

<220>
<223>  /note="Description of artificial sequence: Sox2 forward primer"

<400>  3
gggtgcaaaa agaggagagt ag                                                22


<210>  4
<211>  22
<212>  DNA
<213>  artificial sequence

<220>
<223>  /note="Description of artificial sequence: Sox2 reverse primer"

<400>  4
gcgcctaacg taccactaga ac                                                22


<210>  5
<211>  24
<212>  DNA
<213>  artificial sequence

<220>

<223>  /note="Description of artificial sequence: c-Myc forward primer"

<400>  5
cagaggagga acgagctgaa gcgc                                              24


<210>  6
<211>  27
<212>  DNA
<213>  artificial sequence

<220>
<223>  /note="Description of artificial sequence: c-Myc reverse primer"

<400>  6
ttatgcacca gagtttcgaa gctgttc                                           27


<210>  7
<211>  24
<212>  DNA
<213>  artificial sequence

<220>
<223>  /note="Description of artificial sequence: Nanog forward primer"

<400>  7
agggtctgct actgagatgc tctg                                              24


<210>  8
<211>  24
<212>  DNA
<213>  artificial sequence

<220>
<223>  /note="Description of artificial sequence: Nanog reverse primer"

<400>  8
caaccactgg tttttctgcc accg                                              24


<210>  9
<211>  22
<212>  DNA
<213>  artificial sequence

<220>
<223>  /note="Description of artificial sequence: Dazl forward primer"

<400>  9
gcactcagtc ttcatcagca ac                                                22


<210>  10
<211>  22
<212>  DNA
<213>  artificial sequence

<220>
<223>  /note="Description of artificial sequence: Dazl reverse primer"

<400>  10
ctatcttctg cacatccacg tc                                                22


<210>  11
<211>  22

<212>  DNA
<213>  artificial sequence

<220>
<223>  /note="Description of artificial sequence: Stra8 forward primer"

<400>  11
ccagtctgat atcacagcct ca                                              22


<210>  12
<211>  22
<212>  DNA
<213>  artificial sequence

<220>
<223>  /note="Description of artificial sequence: Stra8 reverse primer"

<400>  12
ttccttgacc tcctctaagc tg                                              22


<210>  13
<211>  22
<212>  DNA
<213>  artificial sequence

<220>
<223>  /note="Description of artificial sequence: Tex18 forward primer"

<400>  13
ggggagggag tagtacctgt tt                                              22


<210>  14
<211>  22
<212>  DNA
<213>  artificial sequence

<220>
<223>  /note="Description of artificial sequence: Tex18 reverse primer"

<400>  14
ccacaccctg gatacttcac tt                                              22


<210>  15
<211>  22
<212>  DNA
<213>  artificial sequence

<220>
<223>  /note="Description of artificial sequence: Fig-a forward primer"

<400>  15
ggagcgaagg tctcagagaa ac                                              22


<210>  16
<211>  22
<212>  DNA
<213>  artificial sequence

<220>
<223>  /note="Description of artificial sequence: Fig-a reverse primer"

<400>  16
tggtagctgt acaatggctc ac                                              22

<210> 17
<211> 22
<212> DNA
<213> artificial sequence

<220>
<223> /note="Description of artificial sequence: Piwil2 forward primer"

<400> 17
cctcctgtaa ctgggaactt gg 22


<210> 18
<211> 22
<212> DNA
<213> artificial sequence

<220>
<223> /note="Description of artificial sequence: Piwil2 reverse primer"

<400> 18
gcaccacaac accctactat ga 22


<210> 19
<211> 24
<212> DNA
<213> artificial sequence

<220>
<223> /note="Description of artificial sequence: Rex1 forward primer"

<400> 19
caccatccgg gatgaaagtg agat 24


<210> 20
<211> 24
<212> DNA
<213> artificial sequence

<220>
<223> /note="Description of artificial sequence: Rex1 reverse primer"

<400> 20
accagaaaat gtcgctttag tttc 24


<210> 21
<211> 22
<212> DNA
<213> artificial sequence

<220>
<223> /note="Description of artificial sequence: Utf1 forward primer"

<400> 21
ctcaaggaca aactccgaga ct 22


<210> 22
<211> 22
<212> DNA
<213> artificial sequence

<220>

<223>  /note="Description of artificial sequence: Utf1 reverse primer"

<400>  22
agacttcgtc gtggaagaac tg                                              22


<210>  23
<211>  22
<212>  DNA
<213>  artificial sequence

<220>
<223>  /note="Description of artificial sequence: c-kit forward primer"

<400>  23
cagtgtgtta cgtggtcatg tg                                              22


<210>  24
<211>  22
<212>  DNA
<213>  artificial sequence

<220>
<223>  /note="Description of artificial sequence: c-kit reverse primer"

<400>  24
gtgtagcact caagtcgaaa cg                                              22


<210>  25
<211>  22
<212>  DNA
<213>  artificial sequence

<220>
<223>  /note="Description of artificial sequence: VASA forward primer"

<400>  25
cttgcagaga tgttcagcag ac                                              22


<210>  26
<211>  22
<212>  DNA
<213>  artificial sequence

<220>
<223>  /note="Description of artificial sequence: VASA reverse primer"

<400>  26
ctccaagagc ttgctctctc tc                                              22


<210>  27
<211>  21
<212>  DNA
<213>  artificial sequence

<220>
<223>  /note="Description of artificial sequence: Eras forward primer"

<400>  27
tctgcgtgac cagtgcttgg c                                               21


<210>  28
<211>  21

<212>  DNA
<213>  artificial sequence

<220>
<223>  /note="Description of artificial sequence: Eras reverse primer"

<400>  28
tcttcaggct acagagcagc c                                                21


<210>  29
<211>  30
<212>  DNA
<213>  artificial sequence

<220>
<223>  /note="Description of artificial sequence: Cripto forward primer"

<400>  29
atggacgcaa ctgtgaacat gatgttcgca                                       30


<210>  30
<211>  30
<212>  DNA
<213>  artificial sequence

<220>
<223>  /note="Description of artificial sequence: Cripto reverse primer"

<400>  30
ctttgaggtc ctggtccatc acgtgaccat                                       30


<210>  31
<211>  21
<212>  DNA
<213>  artificial sequence

<220>
<223>  /note="Description of artificial sequence: Esg1 forward primer"

<400>  31
ataagcttga tctcgtcttc c                                                21


<210>  32
<211>  21
<212>  DNA
<213>  artificial sequence

<220>
<223>  /note="Description of artificial sequence: Esg1 reverse primer"

<400>  32
cttgctagga tgtaacaaag c                                                21


<210>  33
<211>  27
<212>  DNA
<213>  artificial sequence

<220>
<223>  /note="Description of artificial sequence: FGF4 forward primer"

<400>  33
cagcgaggcg tggtgagcat cttcgga                                          27

<210>  34
<211>  27
<212>  DNA
<213>  artificial sequence

<220>
<223>  /note="Description of artificial sequence: FGF4 reverse primer"

<400>  34
cttcttggtc cgcccgttct tactgag                                          27


<210>  35
<211>  21
<212>  DNA
<213>  artificial sequence

<220>
<223>  /note="Description of artificial sequence: Zfp57 forward primer"

<400>  35
atcacttgtg ctgccaaaga c                                                21


<210>  36
<211>  21
<212>  DNA
<213>  artificial sequence

<220>
<223>  /note="Description of artificial sequence: Zfp57 reverse primer"

<400>  36
cttctcctcc tggattccat c                                                21


<210>  37
<211>  20
<212>  DNA
<213>  artificial sequence

<220>
<223>  /note="Description of artificial sequence: PUM1 forward primer"

<400>  37
tggaggcggc tataatacta                                                  20


<210>  38
<211>  20
<212>  DNA
<213>  artificial sequence

<220>
<223>  /note="Description of artificial sequence: PUM1 reverse primer"

<400>  38
ttcttttcac ctttgtcgtt                                                  20


<210>  39
<211>  20
<212>  DNA
<213>  artificial sequence

<220>

<223>  /note="Description of artificial sequence: PUM2 forward primer"

<400>  39
tagaatctcg gggaatggga                                                    20


<210>  40
<211>  20
<212>  DNA
<213>  artificial sequence

<220>
<223>  /note="Description of artificial sequence: PUM2 reverse primer"

<400>  40
tggtttattc gctgtcggat                                                    20


<210>  41
<211>  23
<212>  DNA
<213>  artificial sequence

<220>
<223>  /note="Description of artificial sequence: GFR-a forward primer"

<400>  41
actcctggat ttgctgatgt cgg                                                23


<210>  42
<211>  19
<212>  DNA
<213>  artificial sequence

<220>
<223>  /note="Description of artificial sequence: GFR-a reverse primer"

<400>  42
cgctgcggca ctcatcctt                                                     19


<210>  43
<211>  18
<212>  DNA
<213>  artificial sequence

<220>
<223>  /note="Description of artificial sequence: Zfp145 forward primer"

<400>  43
tgctgcccaa ccacaagg                                                      18


<210>  44
<211>  20
<212>  DNA
<213>  artificial sequence

<220>
<223>  /note="Description of artificial sequence: Zfp145 reverse primer"

<400>  44
cgctgaatga gccagtaaat                                                    20


<210>  45
<211>  23

<212>  DNA
<213>  artificial sequence

<220>
<223>  /note="Description of artificial sequence: b-Actin forward
       primer"

<400>  45
cgtgcgtgac atcaaagaga agc                                              23


<210>  46
<211>  24
<212>  DNA
<213>  artificial sequence

<220>
<223>  /note="Description of artificial sequence: b-Actin reverse
       primer"

<400>  46
atctgctgga aggtggacag tgag                                             24


<210>  47
<211>  26
<212>  DNA
<213>  artificial sequence

<220>
<223>  /note="Description of artificial sequence: Oct3/4, 1st. forward
       primer"

<400>  47
gggattttta gattgggttt agaaaa                                           26


<210>  48
<211>  25
<212>  DNA
<213>  artificial sequence

<220>
<223>  /note="Description of artificial sequence: Oct3/4, 1st. reverse
       primer"

<400>  48
ccaccctcta accttaacct ctaac                                            25


<210>  49
<211>  25
<212>  DNA
<213>  artificial sequence

<220>
<223>  /note="Description of artificial sequence: Oct3/4, 2nd. forward
       primer"

<400>  49
tgaggagtgg ttttagaaat aattg                                            25


<210>  50
<211>  24
<212>  DNA
<213>  artificial sequence

<220>
<223>  /note="Description of artificial sequence: Oct3/4, 2nd. reverse
      primer"

<400>  50
aatcctctca cccctacctt aaat                                              24


<210>  51
<211>  24
<212>  DNA
<213>  artificial sequence

<220>
<223>  /note="Description of artificial sequence: Nanog, 1st. forward
      primer"

<400>  51
tttgtaggtg ggattaattg tgaa                                              24


<210>  52
<211>  23
<212>  DNA
<213>  artificial sequence

<220>
<223>  /note="Description of artificial sequence: Nanog, 1st. reverse
      primer"

<400>  52
aaattttaaa caacaaccaa aaa                                               23


<210>  53
<211>  24
<212>  DNA
<213>  artificial sequence

<220>
<223>  /note="Description of artificial sequence: Nanog, 2nd. forward
      primer"

<400>  53
tttgtaggtg ggattaattg tgaa                                              24


<210>  54
<211>  24
<212>  DNA
<213>  artificial sequence

<220>
<223>  /note="Description of artificial sequence: Nanog, 2nd. reverse
      primer"

<400>  54
aaaaaaacaa aacaccaacc aaat                                              24


<210>  55
<211>  25
<212>  DNA
<213>  artificial sequence

<220>
<223>  /note="Description of artificial sequence: Igf2r, 1st. forward
      primer"

<400>  55
gtagagtttt ttgaattttt ttgtt                                              25


<210>  56
<211>  30
<212>  DNA
<213>  artificial sequence

<220>
<223>  /note="Description of artificial sequence: Igf2r, 1st. reverse
       primer"

<400>  56
taaactataa ttctaattat accaaattac                                         30


<210>  57
<211>  27
<212>  DNA
<213>  artificial sequence

<220>
<223>  /note="Description of artificial sequence: Igf2r, 2nd. forward
       primer"

<400>  57
tggtattttt atgtatagtt aggatag                                            27


<210>  58
<211>  24
<212>  DNA
<213>  artificial sequence

<220>
<223>  /note="Description of artificial sequence: Igf2r, 2nd. reverse
       primer"

<400>  58
aaaaattcta taatcaaaac caac                                               24


<210>  59
<211>  26
<212>  DNA
<213>  artificial sequence

<220>
<223>  /note="Description of artificial sequence: H19, 1st. forward
       primer"

<400>  59
taaggagatt atgttttatt tttgga                                             26


<210>  60
<211>  24
<212>  DNA
<213>  artificial sequence

<220>
<223>  /note="Description of artificial sequence: H19, 1st. reverse
       primer"

<400>  60
ccccctaata acatttataa cccc                                               24

<210>  61
<211>  25
<212>  DNA
<213>  artificial sequence

<220>
<223>  /note="Description of artificial sequence: H19, 2nd. forward
       primer"

<400>  61
aaggagatta tgttttattt ttgga                                          25


<210>  62
<211>  26
<212>  DNA
<213>  artificial sequence

<220>
<223>  /note="Description of artificial sequence: H19, 2nd. reverse
       primer"

<400>  62
aaacttaaat aacccacaac attacc                                         26

## Description

The present invention relates to a method of generating pluripotent stem cells comprising the steps: (a) plating germline stem cells (GSCs) at a density of 20,000 cells or less per 1.9 cm²; and (b) culturing the germline stem cells for at least 11 days without subculturing while maintaining said germline stem cells in an undifferentiated state, wherein during or after step (b) the presence of markers characteristic of pluripotent stem cells is indicative of the generation of pluripotent stem cells. Also, the invention relates to a pluripotent stem cell obtainable by the method of the invention and a cell obtainable by differentiation of said pluripotent stem cell. Further, the invention relates to a method of generating a transgenic non-human animal and a transgenic non-human animal generated by said method. Finally, the invention envisions a composition comprising a pluripotent stem cell of the invention.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Germline stem cells (GSCs) play a key role in the initiation and maintenance of spermatogenesis in the testis. GSCs can be isolated and propagated *in vitro* (Kanatsu-Shinohara, M. et al., Biol Reprod 69, 612-6 (2003); Kubota, H., Avarbock, M. R. & Brinster, R. L., Proc Natl Acad Sci U S A 101, 16489-94 (2004)). An initial report demonstrated the generation of pluripotent cells from postnatal day (PND) 0-2 testicular cells, but not from the adult testis (Kanatsu-Shinohara, M. et al., Cell 119, 1001-12 (2004)). A subsequent report showed that pluripotent cells can be established from retinoic acid 8 (Stra8)-GFP-positive cells (assumed to be GSCs) that were themselves isolated from an adult testis (Guan, K. et al., Nature 440, 1199-203 (2006)). However, the identity of the Stra8-GFP-positive cells is unclear, as is their ability to simultaneously exhibit unipotent and pluripotent phenotypes. More recently, two independent groups have reported that ESC-like cells could be obtained from established GSC lines from both newborn (Kanatsu-Shinohara, M. et al., Biol Reprod 78, 681-7 (2008)) and adult testes (Seandel, M. et al., Nature 449, 346-50 (2007)). However, these cells were multipotent, as evidenced by their inability for germ cell contribution and germ cell transmission.

Recent studies have shown that induced pluripotent stem (iPS) cells could be obtained from mouse and human somatic cells by introduction of exogenous factors (Takahashi, K. & Yamanaka, S., Cell 126, 663-76 (2006); Nakagawa, M. et al, Nat Biotechnol 26, 101-6 (2008); Yu, J. et al., Science 318, 1917-20 (2007)). Although such studies enhance the understanding of the mechanisms of somatic genome reprogramming into pluripotency, direct clinical application of this approach is impeded due to the genomic modifications. Thus, generation of pluripotent cells using a method that minimizes the degree of modification of the target cell, in particular the target cell's genome, may facilitate the feasibility of a clinically safe application of this approach of generating pluripotent stem cells.

The technical problem underlying the present invention was to identify alternative and/or improved means and methods to generate pluripotent stem cells.

The solution to this technical problem is achieved by providing the embodiments characterized in the claims.

Accordingly, the present invention relates in a first embodiment to a method of generating pluripotent stem cells comprising the steps: (a) plating germline stem cells (GSCs) at a density of 20,000 cells or less per 1.9 cm²; and (b) culturing the germline stem cells for at least 11 days without subculturing while maintaining said germline stem cells in an undifferentiated state, wherein during or after step (b) the presence of markers characteristic of pluripotent stem cells is indicative of the generation of pluripotent stem cells.

The term "generating pluripotent stem cells" refers to the generation of cells that at least with regard to their pluripotency exhibit characteristics of embryonic stem cells (ESCs) which belong to the group of pluripotent stem cells. Said characteristics include unlimited self renewal in vitro, a normal karyotype, a characteristic gene expression pattern including stem cell marker genes like Oct3/4, Sox2, Nanog, alkaline phosphatase (ALP) and stem cell-specific antigen 1, 3 and 4 (SSEA1/3/4), and the capacity to differentiate into any specialized cell type (Hanna, J., et al. (2007), Science 318(5858): 1920-3; Meissner, A., et al. (2007), Nat Biotechnol 25(10): 1177-81; Nakagawa, M., et al. (2007), Nat Biotechnol.; Okita, K., et al. (2007), Nature 448(7151): 313-7; Takahashi, K., et al. (2007), Cell 131(5): 861-72; Wernig, M., et al. (2007), Nature 448(7151): 318-24; Yu, J., et al. (2007), Science 318(5858): 1917-20; Park, I. H., et al. (2008), Nature 451(7175): 141-6). In addition, pluripotent stem cells are characterized by the ability to differentiate into any of the three germ layers, viz. endoderm, mesoderm or ectoderm. The state of the art generation of pluripotent stem cells (termed induced pluripotent stem (iPS) cells) from fibroblast cultures has been described, e.g., in Takahashi, Okita, Nakagawa, Yamanaka (2007) Nature Protocols 2(12). The pluripotency of generated pluripotent stem cells can tested, e.g., by *in vitro* differentiation into neural, glia and cardiac cells and the production of germline chimera mice through blastocyst injection. Human pluripotent stem cell lines generated according to the method of the invention can be analyzed, e.g., through *in vitro* differentiation into neural, glia and cardiac cells and their *in vivo* differentiation capacity can be tested by injection into immunodeficient SCID mice and the characterization of resulting tumors as teratomas. The pluripotent stem cells generated according to the method of the invention are termed "germline pluripotent stem (gPS) cells" for the purpose of the present invention and are referred to accordingly hereinafter.

The term "plating" is used herein to describe the process of applying cells onto a suitable surface for cultivation. The term "culturing" in accordance with the invention describes the process of establishing and maintaining a cell culture, at least for a certain period of time such as several days or weeks. The person skilled in the art is well-aware of the means and methods relating to establishing and maintaining a cell culture such as, for example, media constituents, marker choice and selection, cell quantification and isolation. Corresponding methods are described in a variety of textbooks and scientific papers such as, e.g., "Epithelial Cell Culture Protocols", edited by C. Wise, Vol. 188, ISBN: 978-0-89603-893-6, Humana Press; "Practical Cell Culture Techniques", Boulton et Baker (eds), Humana Press (1992), ISBN 0896032140; "Human Cell Culture Protocols", Gareth E. Jones, Humana Press (1996), ISBN 089603335X (Kanatsu-Shinohara, M. et al., Biol Reprod 69, 612-6 (2003); Evans, M.J. et Kaufman, M.H., Nature 292, 154-156 (1981); Thomson, J.A. et al., Science 282, 1145-1147 (1998)) and exemplarily described in the example section infra. Growth media and other cell culture related material as well as instructions and methods for successfully culturing cells, in particular embryonic stem cells (ESCs) and GSCs, can, for example, be obtained at Sigma-Aldrich or Invitrogen. Generally, cells are grown and maintained at an appropriate temperature and gas mixture, i.e. typically 37°Celsius, 5% CO₂, in growth media (a) as irrigating, transporting and diluting fluid while maintaining intra- and extra-cellular osmotic balance, (b) that provides cells with water and certain bulk inorganic ions essential for normal cell metabolism, (c) which - combined with a carbohydrate, such as glucose - provides the principle energy source for cell metabolism and (d) which provides a buffering system to maintain the medium within physiologic pH range, i.e. cells are kept viable. Growth media contain, for example and without limitation, growth factors, nutrient components, glucose, buffers to maintain pH and antifungizides and antibiotics. The culture media for generating gPS cells are essentially the same as the culture media established for culture of germline stem cells of the corresponding species, which are well-known to the person skilled in the art (cf., e.g., Kanatsu-Shinohara, M. et al., Biol Reprod 69, 612-6 (2003); Kubota, H., Avarbock, M. R. & Brinster, R. L., Proc Natl Acad Sci U S A 101, 16489-94 (2004)). Preferably, a suitable GSC culture medium is a culture medium that maintains GSCs viable, proliferating and, preferably, comprises one or more growth factors selected from the group of glial cell line-derived neurotrophic factor (GDNF), GDNF-receptor, basic fibroblast growth factor (bFGF), epidermal growth factor (EGF) and leukemia inhibitory factor (LIF). Preferably, for culturing murine GSCs GDNF and LIF and for culturing human GSCs GDNF and bFGF are parts of the culture medium (preferably in concentrations given below in Table 7). The above cell culture techniques are part of the common general knowledge as evidenced by the various references above. Exemplarily, the ingredients making up a germline cell culture medium that is preferably used in accordance with the method of the invention to culture the GSCs in step (b) are outlined in Tables 1 to 7 as well as in Example 1.

GSCs may be cultured in any of the commercially available cell culture containers such as, e.g., cell culture flasks and multi-well plates in different sizes. Preferably, the GSCs are plated and cultured in cell culture plates such as, e.g., a 24-well plate (1.9 cm² well), while changing cell culture media every 2 or 3 days and adding about 500 µl fresh media.

**Table 1**

| **Ingredient** | **Concentration Range (mg/L) (about)** |
|---|---|
| Human Serum Albumin | 1,000-15,000 |
| HUMANEX-CYTE® | 1 - 15 |
| Ethanolamine | 1 - 25 |
| Sodium Selenite | 0.0001 - 0.01 |
| Hydrocortisone | 0.003 - 0.07 |
| D, L-Tocopherol | 0.005 - 0.05 |
| Iron Saturated Human Transferrin | 10 - 500 |
| Human Zinc Insulin | 1 - 25 |
| N-acetyl-L-cysteine | 16 - 660 |
| 2-Mercaptoethanol | 2 - 8 |

**Table 2**

| **Ingredient** | **Concentration Range (mg/L) (about)** |
|---|---|
| *Inorganic Salts* | |
| CaCl₂ | 1 - 500 |
| KCL | 1 - 500 |
| KNO₃ | 0.008 - 0.8 |
| MgSO₄ | 10 - 500 |
| NaCl | 3,000 - 9,000 |
| NaHCO₃ | 100 - 4,000 |
| NaH₂ PO₄ .multidot. water | 10 - 750 |
| *Amino Acids* | |
| L-Alanine | 5 - 250 |
| L-Asparagine (free base) | 5 - 150 |
| L-Arginine HCl | 10 - 250 |
| L-Aspartic Acid | 5 - 125 |
| L-Cystine 2 multidot. HCl | 1 - 200 |
| L-Glutamic Acid | 5 - 500 |
| Glycine | 5 - 200 |
| L-Histidine .multidot.HCl.multidot.water | 5 - 200 |
| L-Isoleucine | 5 - 500 |
| L-Leucine | 25 - 500 |
| L-Methionine | 5 - 500 |
| L-Phenylalanine | 5 - 500 |
| L-Proline | 5 - 500 |
| L-Serine | 5 - 500 |
| L-Threonine | 5 - 500 |
| L-Lysine .multidot. HCl | 25 - 500 |
| L-Tryptophan | 2 - 100 |
| L-Tyrosine (disodium salt) | 25 - 500 |
| L-Valine | 5 - 500 |
| *Vitamins* | |
| Biotin | 0.01 - 1.0 |
| D-Ca Pantothentate | 0.05 - 10.0 |
| Choline Chloride | 1 - 150 |
| Folic Acid | 0.1 - 10.0 |
| i-Inositol | 1 - 75 |
| Niacinamide | 0.1 - 10.0 |
| Pyridoxal .multidot. HCl | 0.1 - 10.0 |
| Riboflavin | 0.01 - 2.0 |
| Thiamine .multidot. HCl | 0.1 - 10.0 |
| Vitamin B₁₂ | 0.001 - 5.0 |
| *Other Components* | |
| Human Serum Albumin | 1,000 - 15,000 |
| Na₂SeO₃ | 0.001 - 0.01 |
| D-Glucose | 2,000 - 9,000 |
| Phenol Red | 0.5 - 30 |
| HEPES | 1,000 - 7,000 |
| Sodium Pyruvate | 10 - 300 |
| HUMAN EX-CYTE® | 1 - 15 |
| Ethanolamine | 1 - 25 |
| Hydrocortisone | 0.003 - 0.07 |
| D, L-Tocopherol | 0.005 - 0.05 |
| Iron Saturated Human Transferrin | 10 - 500 |
| Human Zinc Insulin | 1 - 25 |
| N-aceyl-L-cysteine | 16 - 660 |
| 2-Mercaptoethanol | 2 - 8 |

**Table 3**

| **Ingredient** | **Concentration Range (µg/ml) (about)** |
|---|---|
| Human Transferrin (holo) | 5 - 20 |
| Insulin (Bovine) | 5 20 |
| Progesterone | 0.0063 - 0.0252 |
| Putrescine | 16.11 - 64.44 |
| Sodium Selenite | 0.0052 - 0.0208 |

**Table 4**

| **Ingredient** | **Concentration Range (mg/l) (about)** |
|---|---|
| Choline Chloride | 0.1 - 10 |
| Folic Acid | 0.1 - 10 |
| myo-Inositol | 0.2 - 20 |
| Niacinamide | 0.1 - 10 |
| D-Pantothenic Acid • ½ Ca | 0.1 - 10 |
| Pyridoxal. HCl | 0.1 - 10 |
| Riboflavin | 0.01 - 1 |
| Thiamine • HCl | 0.1 - 10 |

**Table 5**

| **Ingredient** | **Concentration Range (mg/l) (about)** |
|---|---|
| KCI | 0.2 - 20 |
| KH₂PO₄ (anhyd) | 0.2 - 20 |
| NaCl | 8 - 800 |
| Na₂HPO₄ (anhyd) | 1.15 - 115 |

**Table 6**

| **Ingredient** | **Concentration Range (mg/l) (about)** |
|---|---|
| L-Alanine | 0.89 - 89 |
| L-Asparagine | 1.32 - 132 |
| L-Aspartic Acid | 1.33 - 133 |
| L-Glutamic Acid | 1.47 - 147 |
| L-Glycine | 0.75 - 75 |
| L-Proline | 1.15 - 115 |
| L-Serine | 1.05 - 105 |

**Table 7**

| **Ingredient** | **Concentration Range (about)** |
|---|---|
| D-(+)-glucose | 2 - 30 mg/l |
| Bovine albumin | 2 - 10 mg/l |
| L-glutamine | 1 - 4mM |
| B-mercaptoethanol | 10 - 100 µM |
| Epidermal growth factor (EGF) | 5 - 40 ng/ml |
| Basic fibroblast growth factor (bFGF) | 5 - 40 ng/ml |
| Glial cell line-derived neurotrophic factor (GDNF) | 5 - 100 ng/ml |
| Leukemia inhibitory factor (LIF) | 100 - 10,000 U/ml |

Culture conditions of germline stem cells as well as pluripotent stem cells usually include the use of feeder cells. The term "feeder cells" is a term well-known in the art and is used herein correspondingly. In detail, the term refers to cells such as, e.g., fibroblasts, that serve as a basal layer for other cells such as, e.g., stem cells, and provide - without being bound to a specific scientific theory or providing a scientific explanation - secreted factors, extracellular matrix, and cellular contacts for the maintenance of the cultured cells in an undifferentiated state. For example, commonly used as feeder cells in the culture of murine and human pluripotent and germline stem cells are mouse embryonic fibroblasts (MEFs). Also envisaged is the use of any type of mouse or human somatic cells as feeder cells. Preferably, testicular somatic cells are used as feeder cells to culture GSCs. Feeder cells are plated prior to plating the target cells, i.e. in the present case prior to plating GSCs in step (a). Once plated, feeder cells are generally allowed to grow to 50% to 100% confluency prior to plating of the GSCs. As is well-known in the art, feeder cells can be replaced by any means that mimics the function of feeder cell, referred to herein as "substitutes" of feeder cells. Exemplarily, feeder cells can be substituted by coating the surface of the support the cells are to be plated on with laminin (laminin-coating) at a concentration of 1 to 10 µg/ml (Kanatsu-Shinohara et al., 2005). Once gPS cells, i.e. pluripotent stem cells, have been generated according to the method of the invention, it is preferred that they be transferred into ESC-culture medium and allowed to grow in ESC-culture conditions. ESC-culture conditions as well as ESC-culture media are well-known to the person skilled in the art and have also been described, e.g., in Evans, M.J. et Kaufman, M.H., Nature 292, 154-156 (1981); Thomson, J.A. et al., Science 282, 1145-1147 (1998). Alternatively, gPS cell may be stored, e.g., frozen.

Further, it is to be understood in accordance with the method of the invention that, based on the common general knowledge, the skilled person is in the position to potentially fine tune culture conditions to adapt general cell culture conditions to his specific needs should it become necessary.
The term "germline stem cell" (GSC) is well-known to the person skilled in the art and has the same meaning as used herein. The term "GSC" can also be used interchangeably with the term "SSC" (spermatogonial stem cell). GSCs in accordance with the present invention refer to adult stem cells isolated from the testis, preferably an adult testis. GSCs are unipotent cells incapable of fertilising an egg cell but can give rise to cells that develop into sperm and that produce viable offspring. Isolated GSCs can be cultured for a prolonged time period without losing their properties and can efficiently repopulate the testes of suitable recipient male animals (Oatley, J.M. et Brinster, R.L., Methods Enzymol. 419:259 (2006)). The germline stem cells to be used in the method of the invention can be derived from existing cells lines or obtained by various methods including, for example, obtaining tissue samples in order to establish a primary cell line. Methods to obtain samples from various tissues and methods to establish primary cell lines are well-known in the art (Jones GE, Wise CJ., Methods Mol Biol., 75:13-21 (1997). For example, the germline stem cells may be obtained as outlined in the example section (cf. Example 1) by obtaining testicular cells and subsequent identification and separation of the germline stem cells in cell culture.

In accordance with the method of the invention the germline stem cells are to be plated at a specific density. The density at which the germline stem cells are plated affects the generation of gPS cells, i.e. the conversion rate of GSCs to gPS cells. Without whishing to be bound to a scientific theory, a variety of factors may contribute to said conversion rate such as, e.g. cell-cell adhesion or nutrient depeletion. As a consequence, the number of GSCs plated in step (a) may not be more than 20,000 cells per 1.9 cm² such as, e.g., not more than 17,500; 15,000; 12,500 or not more than 10,000 GSCs per 1.9 cm². More preferred are plating densities in the range of 500 to 4,000 cells per 1.9 cm² such as, e.g., about (for each of the following numerical values) 600; 700; 800; 900; 1,000; 1,200; 1,400; 1,600; 1,800; 2,000; 2,200; 2,400; 2,600; 2,800; 3,000; 3,200; 3,400; 3,600; 3,800 or 4,000 cells per 1.9 cm². Most preferred is a plating density of about 4,000 cells per cm². The term "about" as used herein refers to a deviation from a given value of up to 20%. Also envisaged is plating GSCs at a density of not more than 9,000; 8,000; 7,000; 6,000; 5,000; 4,000; 3,000; 400; 300; 200; 100; 90; 80; 70; 60; 50; 40; 30; 20; 10; 9; 8; 7; 6; 5; 4; 3; 2 or 1 GSC per 1.9 cm². In general, any deliberate number of GSCs in the range of 1 to 20,000 per 1.9 cm² may be plated. The person skilled in the art is in the position to calculate the corresponding cell numbers required when plating cells on an area less or more than 1.9 cm².

The term "subculturing" is another term well-known in the art and in accordance with the present invention has the same meaning. In brief, "subculturing" describes the process of dividing an existing cell culture and subsequently establishing separate subcultures derived therefrom. Generally, this process is performed when cells reach a certain density and must be passaged to keep cells in a proliferative state. In accordance with the invention, the germline stem cells have to be cultured without subculturing for at least 11 days. Since GSCs are to be cultured at least for 11 days culturing times such as, e.g., at least 12, 13, 14, 15, 16, 17, 18, 19, 20 or at least 21 days are envisaged. Preferably, the culturing time is at least 12 to at least 13 days, most preferred at least 14 days. While the presence of gPS cells could be observed as early as 11 days of culture, best results were achieved after about 14 days of culturing the GSCs. Also, culturing without subculturing for more than three weeks such as 4 weeks, 5 weeks, 6 weeks, 7 weeks or 8 weeks is envisioned in accordance with the invention.

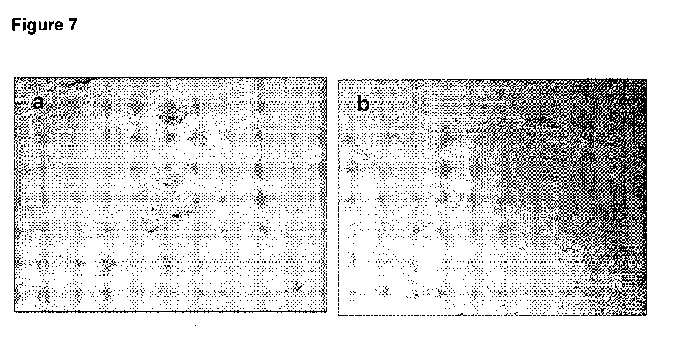

The appearance of gPS cell generation can be determined based on morphology, on molecular and/or functional alterations when compared to the corresponding characteristics of GSCs. The conversion of GSCs to gPS cells is accompanied by a profound change in cell morphology. While GSCs in cell culture exhibit grape-like colonies, gPS cells are displaying an ESC-like morphology which is well-known to the skilled person and distinct to the GSC colonies. Further, gPS cells can aggregate to form embryoid bodies as ES cells. Figure 7 exemplarily shows the morphology of GSCs and gPS cells derived therefrom. Methods to assess the morphology of a cell are well-known in the art and include, e.g., a variety of microscopy techniques such as light microscopy that is a state of the art technique to assess the morphology of cells in culture. Further and preferably, the conversion of GSCs to gPS cells, hence their appearance, is detected using a variety of molecular markers that are unique to pluripotent stem cells, in other words "markers characteristic of pluripotent stem cells", such as, e.g., ESCs. The detection of molecular markers may vary depending on the marker to be detected. The person skilled in the art it well-aware of suitable detection methods for a target marker. Various detection methods and assays are described in, e.g., "Current protocols in Immunology", Wiley Interscience, Print ISSN: 1934-3671, online ISSN: 1934-368X. For example, the transcript level of a marker may be determined. Techniques for the determination of the transcript level include, but are not limited to RT-PCR and its various modifications such as qRT-PCR (also referred to as Real Time RT-PCR). PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2 µl of 10 mM dNTPs, 1 µl total RNA, 10 Units of AMV reverse transcriptase, and H₂O to 20 µl final volume. The reaction may be, for example, performed by using the following conditions: The reaction is held at 70C° for 15 minutes to allow for reverse transcription. The reaction temperature is then raised to 95C° for 1 minute to denature the RNA-cDNA duplex. Next, the reaction temperature undergoes two cycles of 95°C for 15 seconds and 60C° for 20 seconds followed by 38 cycles of 90C° for 15 seconds and 60C° for 20 seconds. Finally, the reaction temperature is herd at 60C° for 4 minutes for the final extension step, cooled to 15C°, and held at that temperature until further processing of the amplified sample. Any of the above mentioned reaction conditions may be scaled up according to the needs of the particular case.

Real-time PCR can, e.g., employ a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SYBR Green for monitoring the de novo synthesis of double stranded DNA molecules.

Further, there are numerous immunohistochemistry methods (Immunocytochemical Methods and Protocols. 2nd Edition. Edited by Lorette C. Javois. Humana Press, Totowa, New Jersey) that may be used to visualize and localize, e.g, protein markers of pluripotency. The selection of a suitable method should be based on parameters relating to type of specimen, fixation, sectioning, blocking, controls, staining, sensitivity and are well known to the person skilled in the art.

Fixation can be achieved with standard formalin fixation using a solution, such as, for example, 4% paraformaldehyde in 0.1 M phosphate buffer, 2% paraformaldehyde with picric acid in 0.1 M phosphate buffer, PLP fixative (4% paraformaldehyde, 0.2% periodate and 1.2% lysine in 0.1 M phosphate buffer) or 4% paraformaldehyde with 0.05% glutaraldehyde. Alternatively, tissues can be rapidly frozen in liquid nitrogen and cut with a cryostat with subsequent fixation with cold acetone or alcohol. In preparation for sectioning the tissue sample can be embedded with paraffin wax or, alternatively frozen tissue can used as stated above. To enhance specific antibody binding or when using multiple antibodies for demonstration of several targets in formalin-fixed, paraffin-embedded tissue sections can be pretreated with antigen retrieval reagents such as heat for varying lengths of time and retrieval solution such as, for example, citrate buffer pH 6.0, TRIS-EDTA pH 9.0 or EDTA pH 8.0. Proteolytic treatment with proteinase K, trypsin, chymotrypsin, pepsin, pronase and various other proteases can also be used to restore immunoreactivity. Moreover, a combination of heat and proteolytic treatment can be used especially when double or triple labeling for two or three antigens co-localized. On frozen or vibratome sections Triton X-100 is a widely used detergent. Other methods to unmask antigens include repeated freezing and thawing or sodium borohydride (1% in phosphate buffer).
Methods for preventing background staining, endogenous peroxidase activity, endogenous alkaline phosphatase activity, endogenous biotin staining or autofluorescence interfering with detection methods are numerous and well known to the person skilled in the art.
Staining can be achieved by direct method involving a labeled antibody reacting directly with the antigen in tissue sections or an indirect method involving a unlabeled primary antibody which reacts with the antigen, and a labeled secondary antibody which reacts with the primary antibody. The latter method providing the advantage of an amplification of the detectable signal due to binding of multiple secondary antibodies to one primary antibody. Other methods include the peroxidase anti-peroxidase method, avidin-biotin complex method, labeled streptavidin biotin method and polymeric methods such as, for example, EnVision System (DakoCytomation), ImmPRESS polymerized reporter enzyme staining system (Vector). Moreover, CSA methods from DakoCytomation providing very high levels of sensitivity and electron microscopic immunohistochemical techniques can be employed to visusalize antibody-antigen binding.

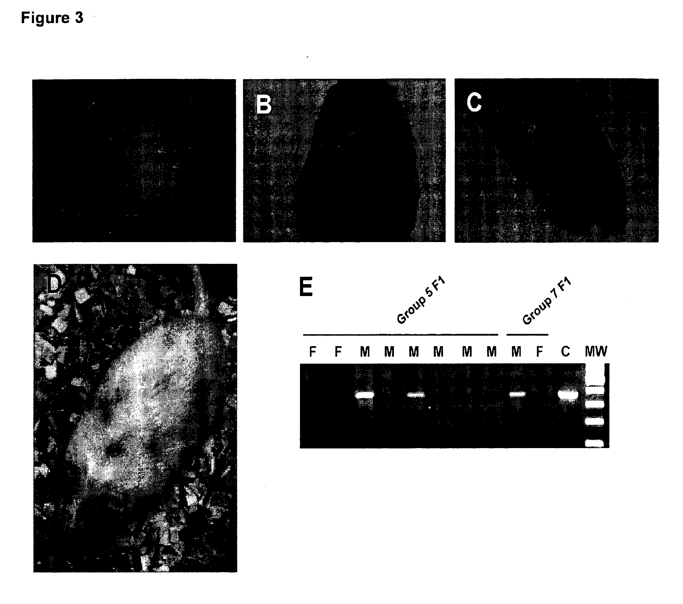

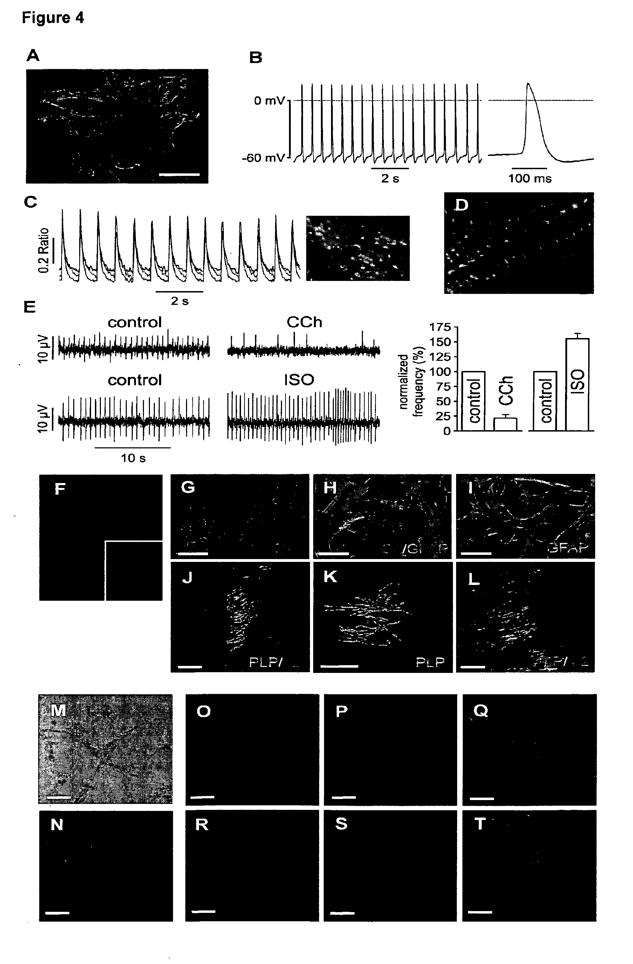

Suitable markers and methods have been described herein above and are further well-known to the person skilled in the art and allow a direct and unambiguous identification of a pluripotent stem cell, i.e. a gPS cell, and also of GSCs that have not been converted into a pluripotent state. The markers to be used may vary from species to species. Preferably, if the appearance or presence of a gPS cell within the GSCs is to be determined on the molecular level, immunostaining with anti-SSEA1, anti-SSEA3 or anti-SSEA4 antibodies (SSEA1 is highly expressed on the surface of murine pluripotent stem cells, while SSEA3 and 4 are highly expressed on the surface of human pluripotent stem cells) may be used and subsequently detected, e.g., via fluorescence activated cell sorting (FACS) analysis. FACS analysis is well-known to the skilled person and also described herein below. Further markers for human gPS cells include but are not limited to TRA-1-60, TRA-1-81 and TRA-2-49/6E whose presence can be analyzed by equally via immunostaining. Also, detection of expression of Nanog, Fgf4, Eras, Rex1, Zfp42, Utf1 or Dppa5a is indicative of the pluripotency of a gPS cell according to the invention. Each marker can be assessed for on its own to determine the appearance or presence of a gPS cell within GSC colonies. Of course, combinations of two or more markers may also be used such as, e.g., Nanog and SSEA1, SSEA3 or SSEA 4 (depending on the origin of the GSCs, cf. above). Alternatively, for the determination of the appearance of gPS cell colonies an alkaline phosphatase (ALP) (ES cell characterization kit (Chemicon)) staining can be used or a chimera assay may be performed (cf. Example 11). The appearance of gPS cells may also be determined by taking advantage of the pluripotency of pluripotent stem cells, hence their capacity to contribute to the three different germlayers (ectoderm, mesoderm and endoderm) in animals with bilateral symmetry when injected into a blastocyst or their capacity to differentiate into specific celltypes (cf. Examples 8 to 11; Fig. 3 and 4). Briefly, a cell whose pluripotency is to be determined is labeled so that cells derived therefrom by cell division and/or differentiation remain detectable, e.g., by incorporation of a GFP-expression construct into the genomic DNA of said cell, and after development of the blastocyst into another embryonic stage or when the fetus is born, the origin of cells can be determined, e.g., by visualizing GFP-expression (cf. Fig. 3 and 4), and hence, the contribution to the germline can be determined.
The terms "during or after" refer to the time point in relation to the culture time given in step (b) when the presence of markers characteristic of pluripotent stem cells may be determined. "During" refers to any time point in the range of 1 to 11 days, i.e. the presence of said markers may be determined on the first day GSCs have been in culture as well as on the eleventh day in culture. The term "after" refers to determining the presence of said markers subsequent to culturing GSCs in step (b) for eleven days, i.e. the first time point being on day 12 after plating GSCs. Accordingly, determining the presence of said markers can be performed at any time subsequent to 11 days of culture as defined in step (b). Methods mentioned throughout this specification and in the prior art may be used to determine pluripotency of a cell during or after step (b).

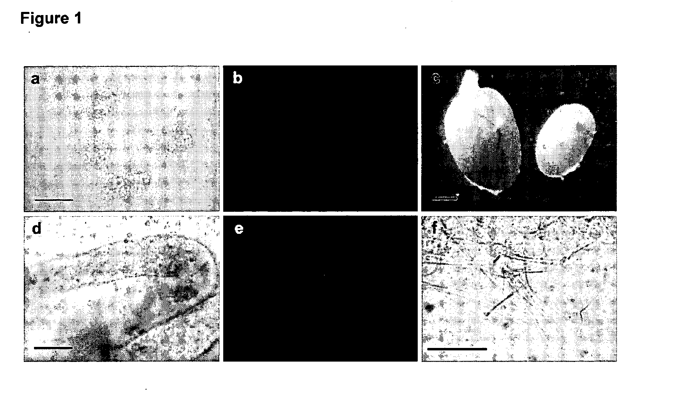

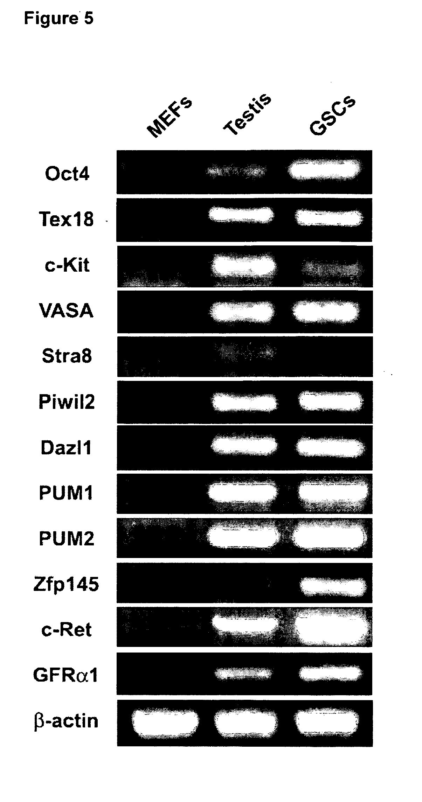

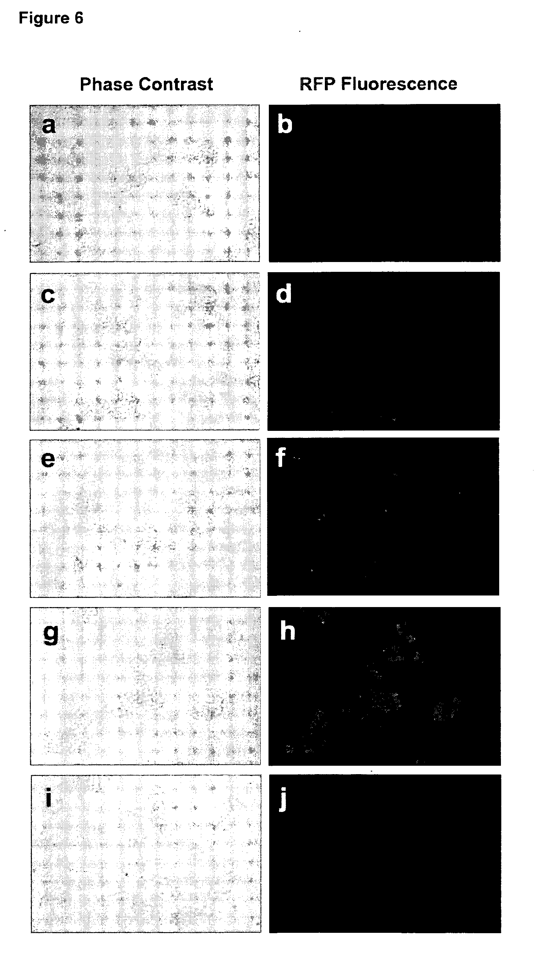

Presently, the state of the art methods to generate pluripotent stem cells exclusively focus on the viral introduction of certain transcription factors that induce the dedifferentiation of target cells into pluripotent stem cells. Surprisingly, it could be demonstrated for the first time that pluripotent stem cells can be generated without the introduction of exogenous factors. Specifically, it could be shown that pluripotent stem cells can be obtained from germline stem cells (GSCs) originally established from an adult testis without viral induction of transcription factors. In detail, a GSC line was established from the testis of PND (postnatal day) 35 Oct4-GFP transgenic mice (OG2) (Szabo, P. E., Hubner, K., Scholer, H. & Mann, J. R., Mech Dev 115, 157-60 (2002)). Expression of a GFP gene under the control of the Oct4 promoter is an indication of Oct4 expression in GSCs, as Oct4 is a GSC-specific marker gene (Kehler, J. et al., EMBO Rep 5, 1078-83 (2004)). Established GSCs cultured on mouse embryonic fibroblasts (MEFs) were found to form colonies of a typical grape-like morphology and expressed Oct4-GFP (Fig. 1a and 1b). Germ cell-specific genes, including Oct4, were detected by reverse transcriptase polymerase chain reaction (RT-PCR) (Fig. 5). The presence of GSC-specific proteins in the established cell line was confirmed by immunocytochemistry (Fig. 6). To further determine the functionality of the cultured GSCs, GSCs were transplanted into the seminiferous tubules of W/W mice. Three months after injection, the colonization of transplanted GSCs was observed and the restoration of spermatogenesis in W/W mice (Fig. 1c-f), confirming the functionality of the established GSCs.

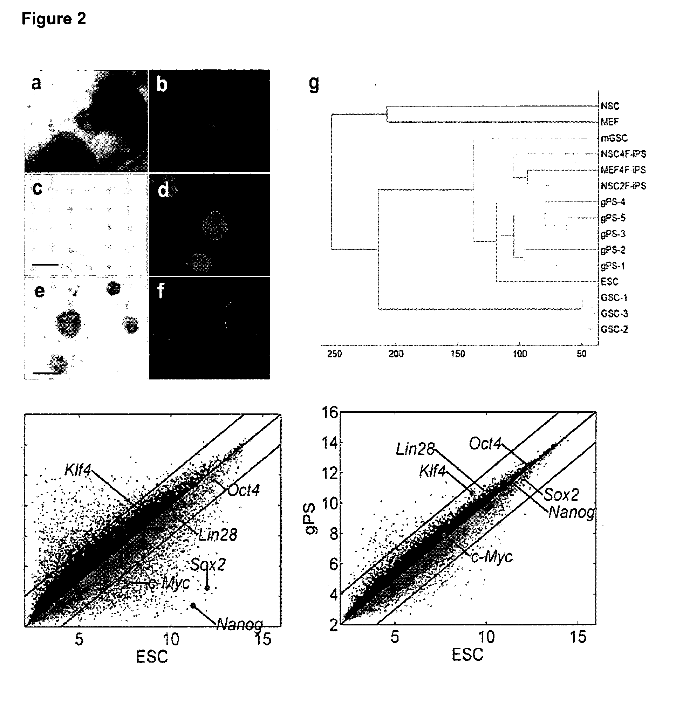

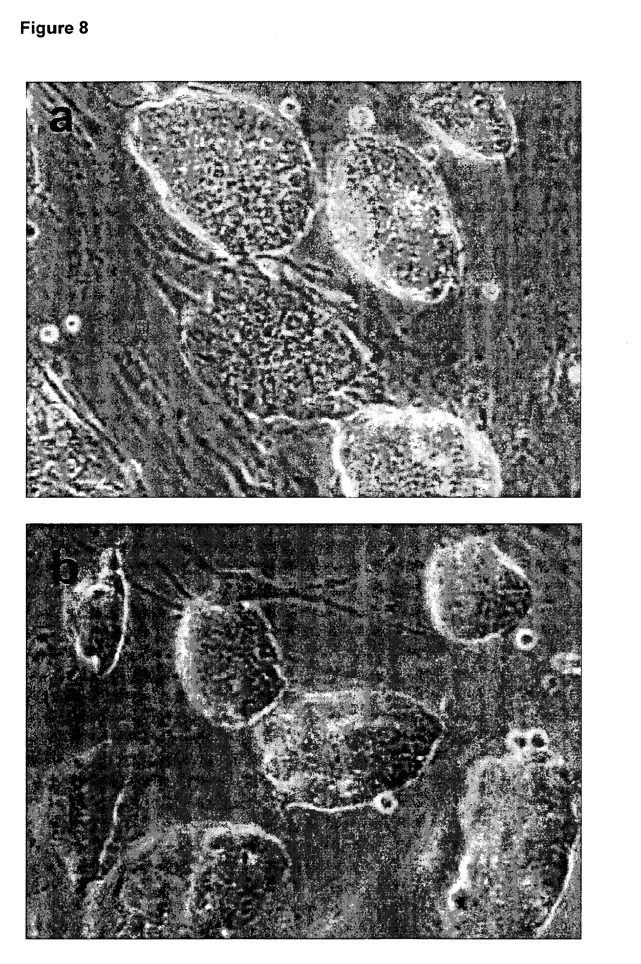

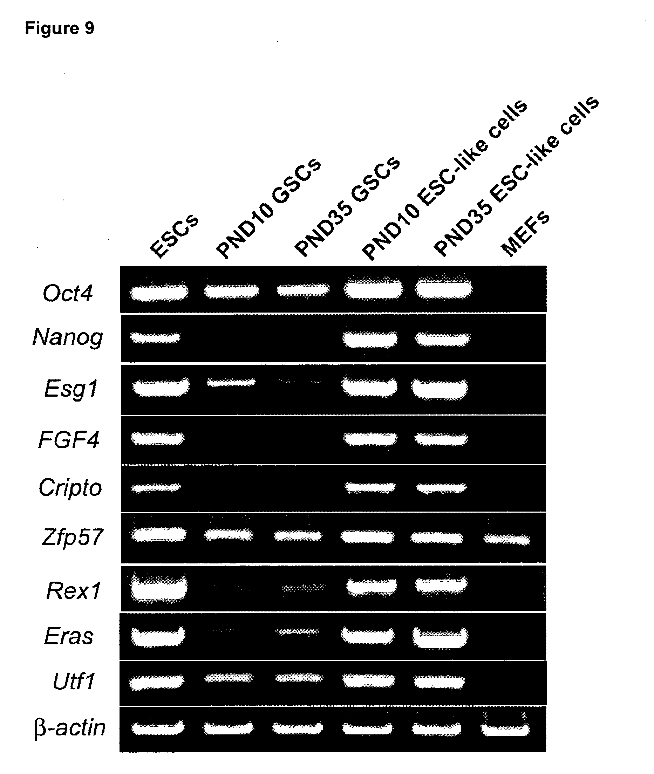

For example, to obtain pluripotent stem cells, approximately 1,000 GSCs were plated per well in 24- well plates containing MEFs. Cultures were maintained in GSC culture medium without subculturing. Within 3 to 4 weeks, colonies with high GFP intensity and a morphology distinct from typical GSC colonies appeared (Fig. 2a and 2b); the GFP-positive colonies were round-shaped, while GSC colonies appear irregular. In repeated experiments using molecular markers instead of morphological markers the presence of gPS cells could be determined as early as 11 days after plating GSCs, i.e. days in culture. To expand the ESC-like cells, colonies were isolated mechanically, dissociated by trypsinisation, and plated onto MEFs in ESC medium (Fig. 2c and 2d). With the same approach, also ESC-like cells from PND 10 (Fig. 7a and 7b) and PND 55 GSCs could be obtained. ESC-like cell colonies appeared in about 10% of the wells, and the cells were maintained under ESC culture conditions. The morphology of ESC-like cell colonies was comparable to that of ESCs (Fig. 8). ESC-like cells expressed high levels of Oct4-GFP and stained positive for alkaline phosphatase (Fig. 2e) and SSEA-1 (Fig. 2f). The expression of genes specific to ESCs by RT-PCR was examined (Fig. 9). The gene expression pattern of ESC-like cells derived from GSCs was similar to that of ESCs. Hierarchical cluster analysis revealed that global gene expression of ESC-like cells is more closely related to ESCs in comparison to other reprogrammed pluripotent cells (Fig. 2g). Therefore, these ESC-like cells are termed "germline pluripotent stem (gPS) cells" by the inventors. Scatter plots of DNA microarray analyses highlight the differences between GSCs and gPS cells and demonstrate the similarity between gPS cells and ESCs (Fig. 2h). It was also found that gPS cells (16 of 20 counts) had a normal karyotype. These results demonstrate that the cellular and molecular characteristics of the gPS cells are similar to those of ESCs.

Also determined was the *in vitro* and *in vivo* differentiation potential of gPS cells. gPS cells could be differentiated *in vitro* into three germ layers. Teratomas formed in all recipients within 4 weeks of transplantation (3/3). Histological assessment of the teratomas revealed the presence of derivatives of the three embryonic germ layers. Teratomas could not be observed after transplantation of GSCs that had not been dedifferentiated to become gPS cells. Of note, testicular transplantation of GSCs into W/W mice restores spermatogenesis in the absence of teratoma formation. In this study, gPS cells, but not GSCs, supported teratoma formation.

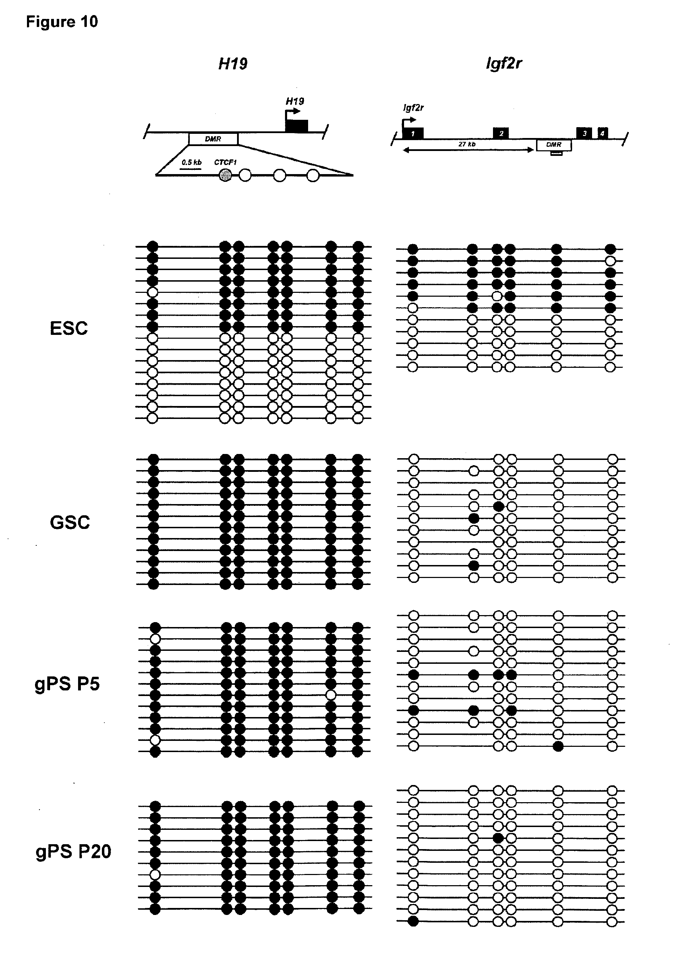

To further confirm the pluripotency of gPS cells, a chimera assay was performed to check the capability of contributing to three germ layers and germ cells using an aggregation protocol. Aggregation was performed with 8-cell-stage embryos and a clump of gPS cells (Fig. 3a). The GFP gene was detected in all three germ layers of E14.5 embryos by genotyping and Oct4 GFP expression in fetal gonads (Fig. 3b and 3c). Furthermore, skin chimerism was found in adult male mice (2/25) (Fig. 3d). Germ cell transmission was confirmed by genotyping of the GFP gene in F1 pups from the chimeric male mice (3/73) (Fig. 3e). Molecular evidence for the origin of gPS cells was provided by the DNA methylation status in differentially methylated regions (DMRs) of paternally imprinted gene H19. To determine the DNA methylation pattern of H19 in three different cell types (GSCs, gPS cells, and ESCs), bisulfite sequencing was conducted (Fig. 10). The data shows that DMRs of H19 in ESCs displayed a heterogeneous imprinting pattern, while those in GSCs show an androgenetic pattern. A previous study showed that the DMRs of H19 in GSCs are fully methylated (Kanatsu-Shinohara, M. et al., Cell 119, 1001-12 (2004)). The androgenetic imprinting pattern of the DMRs of H19 in gPS cells is a particularly , interesting result, as it suggests that the DNA methylation pattern of H19 can be maintained during conversion of GSCs into gPS cells. The same was also shown for the lgf2r gene (cf. also Fig 10).

To further confirm the origin of gPS cells, clonal GSC lines derived from single GSCs were established and further gPS cells generated. The mRNA expression of GSC-specific genes in two clonal GSC lines was analysed (Plate#1Clone#1:P1C1 and P2C3) by RT-PCR and FACS analyses (Fig. 11 and Fig. 12). Also performed was a GSC cluster-forming assay (Yeh, J. R., Zhang, X. & Nagano, M. C., Biol Reprod 77, 897-904 (2007)), an alternative method of assessing the functionality of the cloned GSCs. gPS cells from cloned GSCs expressed pluripotent marker genes (Fig. 13). The chimera assay was then used to assess whether the gPS cells can contribute to the development of germ cells in E13.5 fetal gonads (2/19), and hence exhibit pluripotency. Overall, these results demonstrate that one cell can give rise to a GSC line from which gPS cells can be derived.

To examine the functionality of *in vitro* differentiated cells from gPS cells, gPS cell-derived cardiomyocytes were used. gPS cells were differentiated into α-actinin-positive cross-striated cardiomyocytes (Fig. 4a), which contracted spontaneously with 1.45±0.13 Hz (n=8). The cardiomyocytes displayed action potentials as revealed by intracellular recordings (n=5, Fig. 4b). Ca²⁺ transients of different cardiomyocytes in a beating cluster were synchronized, indicating electrical coupling (Fig. 4c); this was supported by positive connexin 43 staining (Fig. 4d). The chronotropy of gPS cell-derived cardiomyocytes was modulated by hormones of the autonomous nervous system: the muscarinergic agonist CCh reduced frequency to 22±6% (n=4), while the adrenoceptor agonist ISO increased frequency to 155±8% (n=7, Fig. 4e). gPS cells were also capable of differentiating into PECAM-positive endothelial cells (Fig. 4f).

The glial differentiation potential of gPS cells was determined using a previously established protocol (Brustle, O. et al., Science 285, 754-6 (1999); Glaser, T., Perez-Bouza, A., Klein, K. & Brustle, O., Faseb J 19, 112-4 (2005)). The differentiated cells expressed 04 (oligodendrocytes): 33±6%) and GFAP (astrocytes: 55±10%) (Fig. 4g-i). To study whether gPS cell-derived glial precursors are capable of forming myelin *in vivo,* these cells were transplanted into the brain of 2 to 3-day-old myelin-deficient (md) rats. Md rats develop severe CNS hypomyelination due to a point mutation in the X-linked proteolipid protein (PLP) gene and serve as an animal model for Pelizaeus-Merzbacher disease (Koeppen, A. H., Barron, K. D., Csiza, C. K. & Greenfield, E. A., J Neurol Sci 84, 315-27 (1988); Boison, D. & Stoffel, W., Embo J 8, 3295-302 (1989)). Due to the lack of endogenous myelin formation and the absence of PLP expression in md rats, donor-derived internodes can be easily detected by PLP immunolabelling (Duncan, I. D., Grever, W. E. & Zhang, S. C., Mol Med Today 3, 554-61 (1997)). Following injection into the cerebral hemispheres, donor cells were found to propagate in the presence of FGF2 and PDGF in several fibre tracts, including corpus callosum, fimbria, and axon bundles in the septum where they generated parallel PLP-positive profiles characteristic of myelinating oligodendrocytes (Fig. 4j and 4k). Regions with PLP-positive cells also contained donor-derived astrocytes, which were identified with the mouse-specific M2 antibody (Fig. 4j and 4I).
In conclusion, the present invention successfully addresses several of the known draw-backs of prior art methods for dedifferentiating non-pluripotent cells into pluripotent cells, specifically with regard to the potential therapeutic and research application offered by induced pluripotent stem cells. In particular, the omission of the step of viral integration of transgenes required by methods to generate pluripotent stem cells prior to the present invention can be seen as a breakthrough in the corresponding scientific field.

A further embodiment of the invention relates to a method of method of generating pluripotent stem cells comprising the steps: (a) plating germline stem cells (GSCs) at a density of 20,000 cells or less per 1.9 cm²; and (b) culturing the germline stem cells of (a) for 2 to 7 days and subsequently (ba) transferring and plating said germline stem cells without feeder cells or substitutes thereof; or (bb) transferring and plating said germline stem cells without feeder cells or substitutes thereof between 2 to 7 days and subsequently transferring and plating said germline stem cells with feeder cells or substitutes thereof; wherein during or after step (b) the presence of markers characteristic of pluripotent stem cells is indicative of the generation of pluripotent stem cells.

The GSCs in step (b) may be cultured for 2 to 7 days such as 2, 3, 4, 5, 6 or 7 days. In step (ba) cells may be cultured until the gPS cells converted from GSCs can be detected by any one of the methods described above. It is preferred, however, that the GSCs are cultured for at least 4, 5, 6, 7, 8, 9, 10, 11, 12, 13 or at least 14 days. Also longer culturing times of up to 3 weeks, 4 weeks, 5 weeks, 6 weeks, 7 weeks or 8 weeks are in accordance with the invention. The culturing time of the GSCs in step (bb) is between 2 to 7 days without feeder cells such as 4, 5, 6 or 7 days. After replating said GSCs on feeder cells or substitutes thereof said GSCs may be cultured until the presence of a gPS cell can be detected. Preferred are, however, culturing times of at least 4, 5, 6, 7, 8, 9, 10, 11, 12, 13 or at least 14 days. Also longer culturing times of up to 3 weeks, 4 weeks, 5, weeks, 6 weeks, 7 weeks or 8 weeks are in accordance with the invention.
This embodiment is a variation of the method of the invention as described supra. Surprisingly, it is shown that culturing of the GSCs in step (b) with varying conditions as regards the use of feeder cells or substitutes thereof increases the conversion rate of GSCs to gPS cells.
In a preferred embodiment, the method of the invention comprises an additional step prior to step (a) of culturing GSCs between 2 to 7 days without feeder cells or substitutes thereof at a density of less than 20,000 cells per 1.9 cm².

In a further variation of the above described variant embodiment of the invention, the method includes a pre-culture step which further enhances the conversion rate of GSCs to gPS cells.

In a further preferred embodiment of the method of the invention, the germline stem cell endogenously expresses Oct3/4 prior to step (a).
"Expression" in accordance with the invention is meant to encompass expression on the RNA- as well as protein-level. Hence, a gene may be expressed only on the RNA-level but not be translated, i.e. also resulting in protein expression. The term Oct3/4 is interchangeably used herein with the term Oct4. Oct3/4 belongs to the family of octamer ("Oct") transcription factors, and plays a role in maintaining pluripotency. The absence of Oct3/4 in cells normally expressing Oct3/4, such as blastomeres and embryonic stem cells, leads to differentiation into other cell lineages, such as trophoblast, ectoderm, mesoderm, or ectoderm. Thus, in embryonic stem cells the expression of Oct3/4 contributes to pluripotency and differentiation potential.

In a more preferred embodiment, the germline stem cell further endogenously expresses Sox2, KIf4, c-Myc and/or LIN28 prior to step (a).

The Sox family of genes is associated with maintaining differentiation potency similar to Oct3/4, although it is associated with multipotent and unipotent stem cells.

KIf4 of the KIf family of genes was initially identified as a factor for the generation of mouse iPS cells and was demonstrated as a factor for generation of human iPS cells.

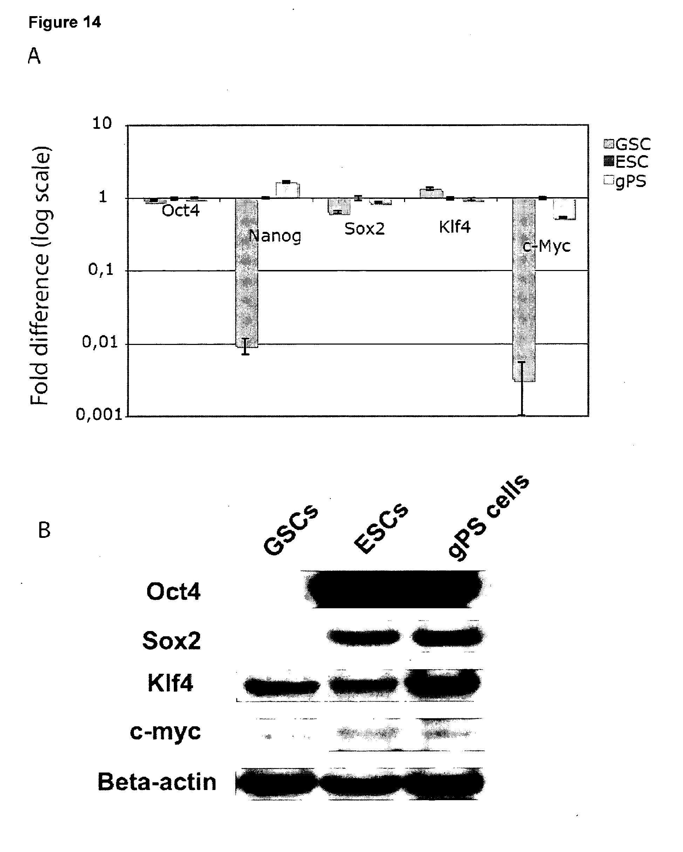

The genes belonging to the Myc family are proto-oncogenes implicated in cancer. It was demonstrated that c-Myc is a factor implicated in the generation of mouse iPS cells and that it was also a factor implicated in the generation of human iPS cells. Introduction of the "Myc" family of genes into target cells for the generation of iPS cells is troubling for the use of iPS cells in clinical therapies, as 25% of mice transplanted with c-Myc-induced iPS cells developed lethal teratomas. N-Myc and I-Myc have been identified to replace c-Myc with similar efficiency. Most preferred in accordance with the invention is that the GSCs endogenously express Oct3/4 and Sox2 - at least on the mRNA level.
For germline stem cells exemplary the expression levels of Oct4, Sox2, KLF4, Nanog and c-Myc were assessed (cf. Fig. 14). On the mRNA level, expression of Oct4, Sox2 and KLF4 are comparable to the expression of said genes in ESCs, while Nanog and c-Myc mRNA levels are lower than their ESC counterparts.

However, on the protein level Oct4 is significantly less expressed than in ESCs. The same is true for Sox2, whose expression could not be detected on the protein level, although being expressed on the mRNA level at a comparable level as in ESCs. In contrast, c-Myc protein expression is comparable to that in ESCs, although c-Myc mRNA expression is significantly lower than in ESCs.

In another preferred embodiment of the method of the invention, the germline stem cells are plated at a density of 500 to 4,000 cells per 1.9 cm² in step (a).

A density of 500 to 4,000 cells per 1,9 cm² has been shown to result in the highest conversion rates of GSCs to gPS cells. For example, at a density of 4000 cells per 1.9 cm² approximately 8 gPS cell colonies could be observed on a 24-well plate.

As outlined above and a preferred embodiment of the method of the invention; the GSCs are obtained by testicular biopsy described in this specification (cf. Example 1).

Also preferred in the method of the invention is that the germline stem cells are rodent, bovine, porcine, non-human primate or human germline stem cells. Of course, a germline stem cell of any other mammalian organism may be used in accordance with the invention.

In a more preferred embodiment, the rodent germline stem cells are murine germline stem cells.

In another embodiment, the invention relates to a pluripotent stem cell obtainable by the method of the invention.

The morphological and functional characteristics of pluripotent stem cells obtainable or obtained by the method of the invention are described herein supra and infra and without being bound to a scientific theory can be compared to ES cells. Surprisingly, the present inventors were able to show that on a genetic level gPS cells can be differentiated from other pluripotent stem cells such as ES cells. Briefly, molecular evidence for the origin of gPS cells was provided by the DNA methylation status in differentially methylated regions (DMRs) of paternally imprinted gene H19 or Igf2r as described herein supra. Hence, pluripotent stem cells obtainable or obtained by the method of the invention are different from other - endogenous or artificially induced - pluripotent stem cells (at least) by way of specific methylation patterns. Further genes whose methylation pattern can be assayed to determine the origin of a pluripotent stem cell are well-known in the art.
A composition comprising the gPS cells of the invention (as well as the gPS cells of the invention per se) can be used in a variety of experimental as well as therapeutic scenarios. The gPS cells of the invention having no transgenic expression elements and an overall reduced risk of developing into cancerous cells are expected to be beneficial in gene therapy, regenerative medicine, cell therapy or drug screening.
Gene therapy, which is based on introducing therapeutic DNA constructs for correcting a genetic defect into germ line cells by *ex vivo* or *in vivo* techniques, is one of the most important applications of gene transfer. Suitable vectors and methods for *in vitro* or *in vivo* gene therapy are described in the literature and are known to the person skilled in the art (Davis PB, Cooper MJ., AAPS J. (2007), 19;9(1):E11-7; Li S, Ma Z., Curr Gene Ther. (2001),1(2):201-26). In accordance with the invention, germline stem cells obtained from a patient could, for example, be genetically corrected by methods known in the art and subsequently be reprogrammed into gPS cells having the pheno- and genotype of ES cells, by the method of the invention. This evidences the applicability of gPS cells in gene therapy and/or cell therapy.
Regenerative medicine can be used to potentially cure any disease that results from malfunctioning, damaged or failing tissue by either regenerating the damaged tissues in vivo or by growing the tissues and organs in vitro and subsequently implanting them into the patient. The gPS cells of the invention being capable of differentiating into virtually any tissue (ectoderm, mesoderm, endoderm cells) can be used in any aspect of regenerative medicine and hence drastically reduce the need for ES cells.
The gPS cells of the invention can also be used to identify drug targets and test potential therapeutics hence reducing the need for ES cells and in vivo studies. Experimental setups and methods to identify and/or assess effects of a potential drug including, for example, target-site and -specificity, toxicity, bioavailability, are well-known to the person skilled in the art.

Further, the gPS cells may be used to study the prevention and treatment of birth defects or study cell differentiation.
Also, the gPS cells of the invention may be useful in experimental settings - besides therapeutic applications - to study a variety of aspects related to dedifferentiation when inducing reprogramming of a germline stem cell such as, e.g., spatiotemporal shifts in the expression pattern of genes or of methylation patterns, or the morphological changes leading to changes in aggregation behaviour. The gPS cells can further be subject to studies relating to, e.g., gene therapy, gene targeting, differentiation studies, tests for safety and efficacy of drugs, transplantation of autologous or allogeneic regenerated tissue, tissue repair (e.g., nervous system, heart muscle), diseases like, e.g., Parkinson's disease, heart attack, diabetes, cancer, leukaemia or spinal cord injury, embryonal gene expression, genetic manipulation of embryonal genes, early embryology and fetal development, identification of embryonic cell markers, cell migration or apoptosis.

In a further embodiment, the invention relates to a cell obtainable by differentiation of the pluripotent stem cell of the invention.

As outlined herein-above, pluripotent cells are characterised by their ability to differentiate into any type of cell. This is evidenced, e.g., by the contribution of pluripotent stem cells to the three germ layers, i.e. mesoderm, endoderm and ectoderm.

Accordingly, the cell obtainable or obtained can be a mesodermal, endodermal or ectodermal cell. As shown herein below (cf. e.g., Example 11, Fig. 3), the gPS cells of the invention can be differentiated into mesodermal, endodermal or ectodermal cells. Conditions to differentiate pluripotent stem cells such as the gPS cells of the invention vary depending on the cell type to be obtained (cf. Brustle, O. et al.. Science 285, 754-6 (1999); Igelmund, P. et al. Pflugers Arch 437, 669-79 (1999)). Exemplarily, conditions for differentiation of the gPS cells of the invention into neural cells, endothelial cells and cardiomyocytes are described herein (cf. Examples 9 and 8, respectively). Cells obtainable or obtained by differentiation of the gPS cells of the invention include cells varying in differentiation status such as, e.g., multipotent (progenitor cells) or terminally differentiated cells. For example, hematopoietic stem cells, mesenchymal stem cells, epithelial stem cells or muscle satellite cells can be obtained. Further, any terminally differentiated cell can be obtained by differentiation of gPS cells of the invention. For example, neural cells (cf. Fig. 4; Example 9), cardiomyocytes (cf. Fig 4, Example 8), and endothelial cells (Fig. 4; Example 14). Cells obtained or obtainable by differentiation are different from the same but endogenously occurring cells at least with regard to the methylation pattern of the imprinting genes H19 and/or Igf2r. Said imprinting pattern is different in, e.g., endogenous cardiomyocytes as compared to cardiomyocytes derived from gPS cells which show the methylation pattern of GSCs. The cells obtainable or obtained by differentiation of the pluripotent stem cells of the invention may be used in a variety of therapeutic and research settings. For, example, a patient can be treated with an autologous transplant generated by obtaining GSCs from said patient, converting the GSCs into pluripotent stem cells according to the method of the invention and subsequent differentiation into any desired cell type intended to be transplanted. Autologous transplants have the benefit of not causing an adverse reaction of the immune system in the transplant recipient such as, e.g., "graft-versus-host disease (GVHD)".

Another embodiment of the invention is directed to a method of generating a transgenic non-human animal comprising the steps of: (a) introducing a pluripotent stem cell according to the invention or a pluripotent stem cell generated according to the method of the invention into an non-human preimplantation embryo; (b) transferring the embryo of step (a) into the uterus of a pseudo-pregnant female non-human animal; and (c) allowing the embryo to develop and to be born.

The term "transgenic non-human animal" as used in accordance with the invention relates to an animal in which there has been effected a deliberate modification of its genome by methods described herein.

The method of the invention of generating a transgenic non-human animal is preferably carried out according to methods that have been established for generating transgenic non-human animals by the use of embryonic stem cells, however, replacing the embryonic stem cells with gPS cells of the invention. Said methods are well-known in the art (Hogan, B., R. Beddington, et al. (1994), "Manipulating the Mouse Embryo: A Laboratory Manual", Cold Spring Harbour Press; Hanna, J., et al. (2007), Science 318(5858): 1920-3; Meissner, A., et al. (2007), Nat Biotechnol 25(10): 1177-81; Nakagawa, M., et al. (2007), Nat Biotechnol.; Okita, K., et al. (2007), Nature 448(7151): 313-7; Takahashi, K., et al. (2007), Cell 131(5): 861-72; Wernig, M., et al. (2007), Nature 448(7151): 318-24; Yu, J., et al. (2007), Science 318(5858): 1917-20; Park, I. H., et al. (2008), Nature 451(7175): 141-6). In brief, introduction of the gPS cell into a non-human preimplantation embryo, like a morula or a blastocyst, is preferably effected by microinjection into a morula or blastocyst or by aggregation of gPS cells with 8-cell or morula embryos. Said chimaeric embryo is then transferred into the uterus of a pseudopregnant non-human female where it develops into an embryo that is finally born (cf. Example 11).

Generating a transgenic non-human animal line from gPS cells is based on the pluripotency of said gPS cells (i. e., their ability, once injected into a host developing embryo, such as a blastocyst or morula, to participate in embryogenesis and to contribute to the germ cells of the resulting animal). As outlined above, the blastocysts containing the injected gPS cells are allowed to develop in the uteri of pseudopregnant non-human females and are born as chimeras. The resultant transgenic non-human animals are chimeric for cells originating from gPS cells and are backcrossed to wildtype non-human animals and screened for animals carrying only the genetic content of an gPS cell so as to identify transgenic animals homozygous for the combination of DNA segments.

The transgenic non-human animals may, for example, be transgenic mice, rats, hamsters, dogs, monkeys, rabbits, pigs, or cows. Preferably, said transgenic non-human animal is a mouse.

Accordingly, the invention also relates to a transgenic non-human animal generated by the method of the invention.

Finally, an embodiment of the invention relates to a composition comprising a pluripotent stem cell according to the invention or a pluripotent stem cell obtainable by the method of invention.

A composition as used herein relates to a composition that comprises gPS cells and preferably further constituents that maintain cell viability of said cells. Such constituents are well-known to the skilled person and comprise, for example, cell media constituents. Further, depending on the intended application the composition may comprise additional constituents, for example, constituents facilitating administration to a patient in the case that the composition is a pharmaceutical composition. In accordance with the present invention, the term "pharmaceutical composition" relates to a composition for administration to a patient, preferably a human patient. The pharmaceutical composition of the invention comprises the compounds recited above. It may, optionally, comprise further molecules capable of altering the characteristics of the gPS cells of the invention thereby, for example, stabilizing, and/or modulating their function. The pharmaceutical composition of the present invention may, optionally and additionally, comprise a pharmaceutically acceptable carrier or diluent. Examples of suitable pharmaceutical carriers are well known in the art and include, e.g., buffered saline solutions, sterile solutions, organic solvents, non-toxic semisolid or liquid filler, encapsulating material or formulation auxiliary of any type. Compositions comprising such carriers can be formulated by well-known conventional methods. The mode of administration of the composition to a patient depends on a variety of factors and may be achieved, e.g., by intravenous, intramuscular, intraperitoneal, intrasternal, subcutaneous and intraarticular injection and infusion or implantation and deposition at or near the target site. The pharmaceutical compositions can be administered at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician.

The figures show:
**Figure 1****: Establishment of a GSC line from the testis of PND35 Oct4-GFP mice** Typical GSC colonies formed in culture. b) GSCs express the GFP gene under control of the Oct4 promoter (same detail as in a). c) Three months after transplantation of GSCs into the W/W recipient mice, the testes were larger than those of controls. d) and e) Photomicrographs of the testicular tubules of a recipient mouse. Note that transplanted Oct4-GFP-positive GSCs (e) colonized the seminiferous tubules. f) The testes of recipient mice produced spermatozoa. Spermatogenesis in the testes of W/W mice was restored by the transplantation of GSCs. Scale bars, 100 µm (a and d), 2 mm (c), 25 µm (f).
**Figure 2****: Establishment of gPS cells and cellular and molecular characterisation of gPS cells**
   a) and b) Conversion of GSCs into ESC-like cells after 4 weeks in culture. Distinct Oct4-GFP-expressing colonies were observed in the induction culture. c) and d) Established ESC-like cells from Oct4-GFP-expressing colonies. The colonies displayed a morphology similar to that of ESC colonies and were positive for Oct4-GFP. e) ESC-like cells stained positive for alkaline phosphatase. f) Immunofluorescence staining revealed SSEA1 expression in. the ESC-like cells. g) Hierarchical analysis between different cell types: ESCs, GSCs, neural stem cells (NSCs), MEFs, gPS cells, NSC-iPS cells , MEFiPS cells, and multipotent GSCs (mGSCs). h) Comparison of global gene expression between ESCs and GSCs (right), and between ESCs and gPS cells (left). Scale bars, 200 µm (a), 100 µm (c and e).
**Figure 3****: Analysis of chimera formation**
   A) Blastocyst stage of an embryo created by the aggregation of an 8-cell-stage embryo with gPS cells. Note that Oct4-GFP-positive gPS cells contributed to the development of the inner cell mass. B) to E) Male (B and C) and female (D and E) fetal gonads from E14.5 embryos. Oct4-GFP-positive germ cells were detected both in male (C) and female gonads (arrowheads in e). F) Arrowhead indicates black coat color chimerism by donor gPS cells (C57BL6 background). G) Genotyping of GFP gene in F1 offspring. M: male, F: female, C: positive control, MW: molecular weight marker.
**Figure 4****: Differentiation and function of gPS cell-derived cardiomyocytes, neural cells, and endothelial cells**
   A) and D) α-actinin-positive (green) cross-striated cardiomyocytes. B) Action potentials recorded with a sharp electrode from a beating area (d5+7 EB); right panel: enlarged single action potential. C) Spontaneous and synchronous Ca²⁺ transients (left panel) recorded from three different (cells are marked in the right panel, ∼ 200-300 µm apart) Fura-2 loaded cardiomyocytes. D) Connexin 43 staining in adjacent cardiomyocytes (red). E) Extracellular field potentials recorded with multielectrode arrays from beating areas of differentiated (d4+14) EBs. Negative (top traces, charbachol: CCh) and positive (lower traces, isoprenaline: ISO) chronotropic modulation is observed. Hormonal modulation statistics of normalized beating frequencies (right panel; p<0.05; CCh: n=4, ISO: n=7, error bar: s.e.m.). F) Clustered PECAM-positive (red) endothelial cells; the inset highlights membrane staining. Gg-li) Following *in vitro* growth factor withdrawal, gPS-glial precursors (GP) produced 33±6% O4-positive oligodendrocytes and 55±10% GFAP-positive astrocytes. Jj-Ll). Upon transplantation of gPS-GP cells into the brain of myelin-deficient (md) rats, myelination of host axons in the corpus callosum was observed. Newly formed internodes are identified using an antibody to PLP, which is absent in the PLP-deficient md rats. Astrocytes are labelled with the mouse-specific M2 antibody. Gg-li, conventional fluorescence photomicrograph; Jj-Ll, confocal image., Scale bar: 50 µm (A), 230 µm (C), 8 µm (D), 100 µm (F), 25 µm (F, insert), 50 µm (G-L).M) Capillary-like structure formation by gPS-derived endothelial cells on matrigel. N) gPS-derived endothelial cells incorporate Dil-conjugated acetylated low density lipoprotein (Dil-Ac-LDL). O-T) Sections of adductor muscles transplanted with β-galactosidase transgenic gPS-derived endothelial cells. β-galactosidase immunofluorescence detection (O, R) and identification of endothelial cells by binding of TRITC-conjugated BS-1 lectin (P, S). Arterial cross (O-Q) and longitudinal (R-T) sections are shown. Scale bar: 50 µm (A), 230 µm (C), 8 µm (D), 100 µm (F), 25 µm (F, insert), 50 µm (G-L), 25 µm (M-T).
**Figure 5****: RT-PCR analysis for expression of GSC specific genes**
   GSC marker gene expression in MEFs, adult testis, and PND35 GSCs was analyzed.
**Figure 6****: Protein expression in established GSCs detected by immunohistochemistry**
   Expression of GSC-specific proteins used in the analysis was confirmed. (a and b) negative control; incubation only with the secondary antibody, (c and d) GFRα1, (e and f) CD9, (g and h) VASA. (i and j) Notch1, whose expression is usually found in differentiated GSCs, was not detected in GSCs.
**Figure 7****: Morphology of established GSCs form PND10 testis and ESC-like cells converted form the GSCs**
   a) PND10 GSCs. B) ESC-like cells converted form PND10 GSCs
**Figure 8****: Morphology of ESC-like cells**
   a) Bright field picture of ESCs. B) Bright field picture of ESC-like cells. The morphology of ESC-like cells is similar to that of ESCs.
**Figure 9****: RT-PCR analysis for pluripotent marker genes**
**Figure 10****: Methylation status of differentially methylated region (DMR) of H19 and imprinting control region (ICR) of Igf2r in ESC, GSC, passage (p) 5 gPS, and p20 gPS**
   DNA methylation was analyzed by bisulfite genomic sequencing. Open and filled circles indicate unmethylated and methylated CpGs, respectively.

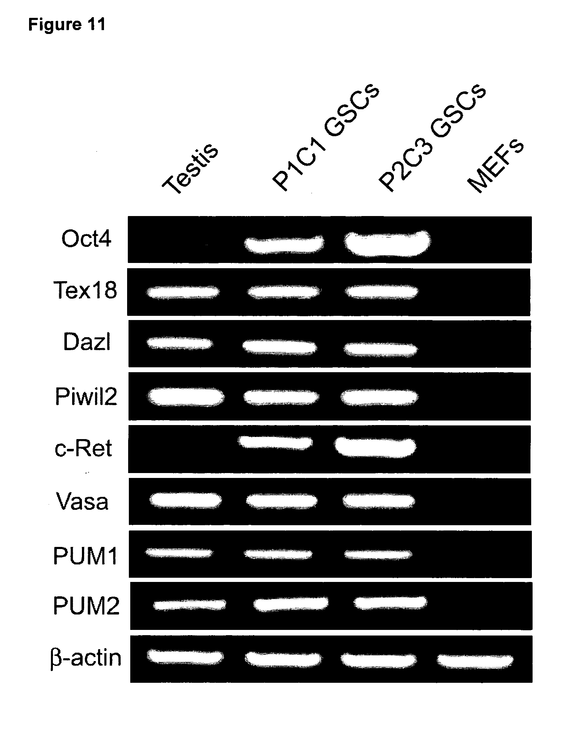

**Figure 11****: RT-PCR analysis for GSC-specific gene expression in clonally established GSC cell lines**
   GSC-specific expression was analyzed in testis, P1C1 GSCs, P2C3 GSCs, and MEFs (as negative control).

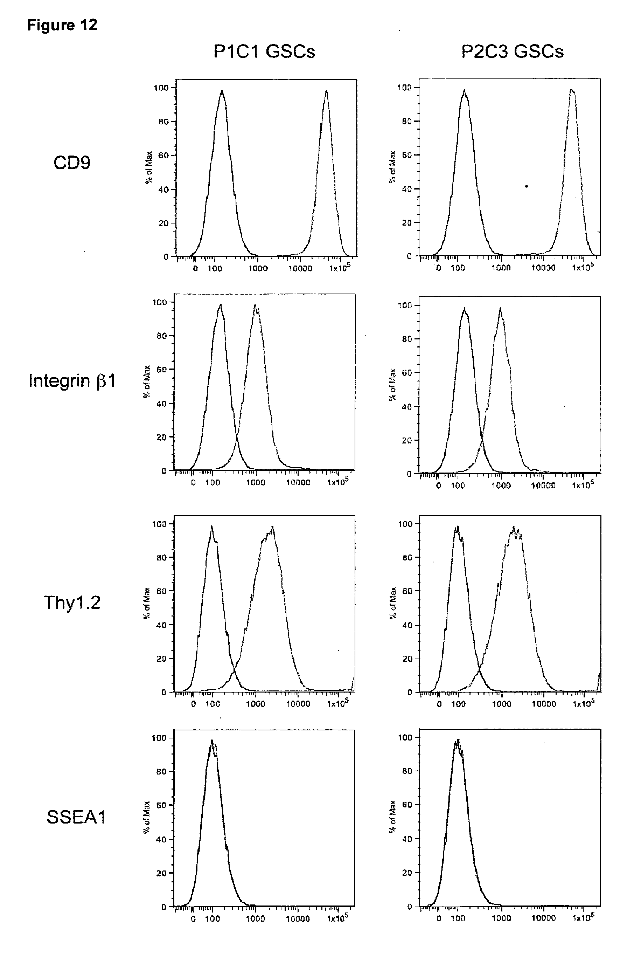

**Figure 12****: Flow-cytometric analysis for GSC-specific surface marker proteins on established GSCs**
   Histograms filled with red color represent GSCs stained with the antibodies indicated. Open histograms indicate cells stained with the isotype control antibody.
   Cultured GSCs were positive for CD9, integrin β1, Thy 1.2, but not for SSEA1, which is an ESC marker.

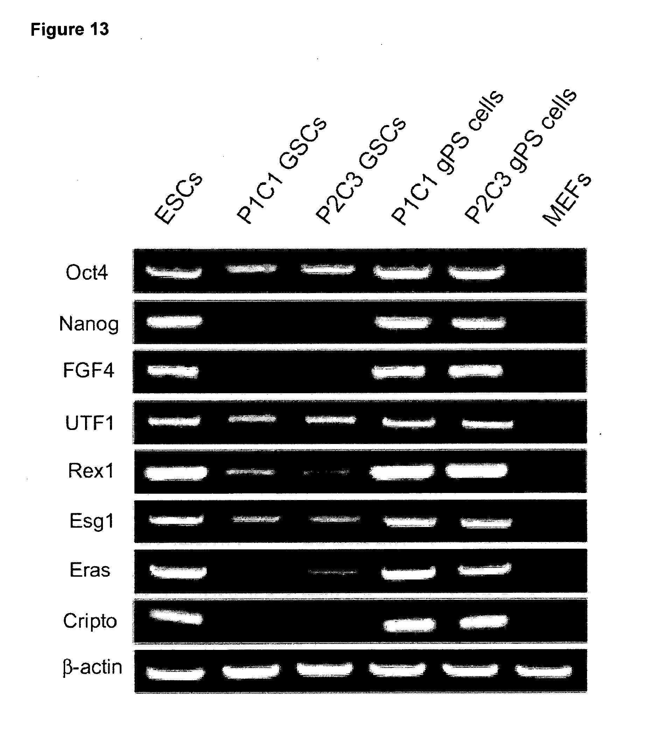

**Figure 13****: RT-PCR analysis of ESC-specific gene expression in gPS cells derived form clonally established GSC lines**
   gPS cells from clonal GSC lines showed similar patterns in ESC-specific gene expression in comparison to ESCs.
**Figure 14****: RT-PCR and Western blot analysis of Oct4, Nanog, Sox2, KIf4 and c-Myc in ESCs, GSCs and gPS cells**
   A. Quantitative RT-PCR analysis shows that Oct4, Sox2, and KIf4 mRNA expressions in spermatogonial stem cells are comparable to those of ESCs while Nanog and c-Myc mRNA expression are lower than those of ESCs. B. Western blot analysis shows that Oct4 protein is expressed in spermatogonial stem cells even though the level is lower than in ESCs. Sox2 protein expression is not detected in GSCs even if Sox2 mRNA is expressed in GSCs at the level of ESCs. C-Myc protein expression in spermatogonial stem cells is still comparable to that of ESCs whereas the mRNA expression level is lower than in ESCs.

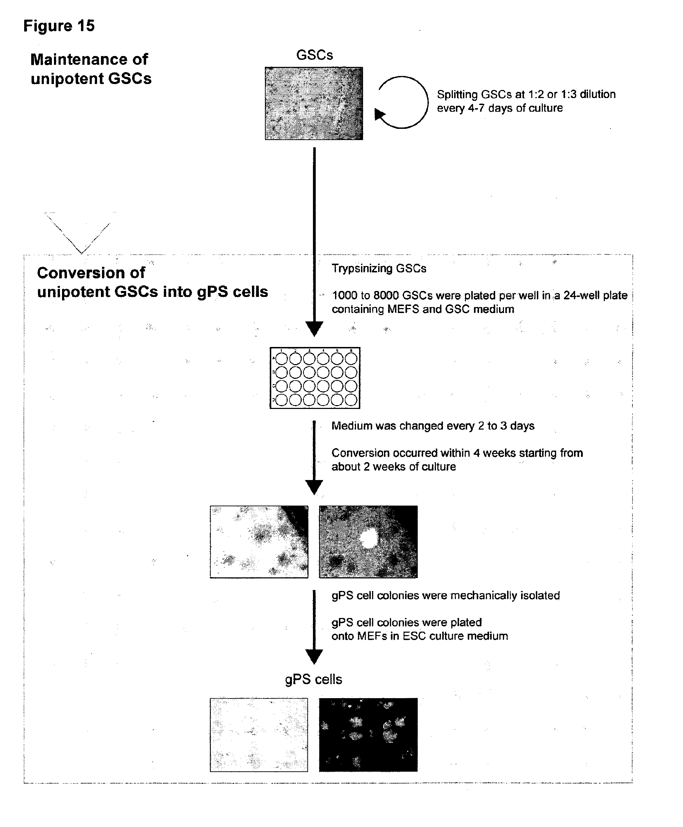

**Figure 15****: Schematic representation of the method to convert GSCs to gPS cells**

The examples illustrate the invention:

### Example 1: Cell culture

Testicular cells were obtained from the testes of Oct4-GFP transgenic (OG2) or OG2/LacZ transgenic mice by a two-step digestion as previously described (Kanatsu-Shinohara et al., 2003). Briefly, whole testis tissue or biopsy testicular tissue (more than 10,000 cells containing testicular tubule) was placed in Digestion solution I, which contained collagenase type IV (1 mg/ml) (Sigma) and DNAse (0.5 mg/ml) (Sigma), in a 37°C water bath. After separation of interstitial cells by gravity for 10 minutes, testicular tubules were washed with DMEM/F12 (Invitrogen) and treated with Digestion solution II, which contained collagenase IV (1 mg/ml) (Sigma), DNAse (0.5 mg/ml) (Sigma), and hyaluronidase (0.5 mg/ml) (Sigma), at 37°C for 10 minutes. The digested tubules were additionally pipetted to obtain a single cell suspension, followed by washing with DMEM/F12 supplemented with 15% FCS and filtering through a 45 mm nylon mesh. Cell numbers were assessed using a Thomas chamber. The suspension of testicular cells in germline stem cell (GSC) culture medium was plated onto 0.2% (w/v) gelatin-coated culture dishes (2 × 10⁵ cells/3.8 cm²). Within 7 to 10 days in culture, GSC colonies were observed under the microscope. GSC colonies were detached by gentle pipetting and replated onto mitomycin C-inactivated or irradiated mouse embryonic fibroblasts (MEFs). The cells were maintained on MEFs and passaged every 4 to 7 days at a dilution of 1:2 to 1:3.
GSC lines were established using the modified GSC medium consisting of StemPro-34 SFM (Invitrogen) supplemented with StemPro supplement (Invitrogen), 1 × N2 supplement (Invitrogen), 6 mg/ml D-(+)-glucose (Invitrogen), 30 mg/ml pyruvic acids (Invitrogen), 1 ml/ml DL-lactic acids (Invitrogen), 5 mg/ml bovine albumin (Invitrogen), 2 mM L-glutamine (Invitrogen), 50 mM β-mercaptoethanol (Invitrogen), penicillin/streptomycin (Invitrogen), minimal essential medium (MEM) vitamins (Invitrogen), MEM nonessential amino acids (Invitrogen), 30 ng/ml b-estradiol (Sigma), 60 ng/ml progesterone (Sigma), 20 ng/ml mouse epidermal growth factor (EGF) (Invitrogen), 10 ng/ml human basic fibroblast growth factor (FGF) (Tebu-bio), 10 ng/ml human glial cell line-derived neurotrophic factor (Tebu-bio), and 10³ U/ml ESGRO (murine leukemia inhibitory factor) (Chemicon) with 1% FBS. ESCs and gPS cells were grown on MEFs in ESC medium (DMEM supplemented with 15% FBS, MEM nonessential amino acids, 2 mM L-glutamine, penicillin/streptomycin, 50 mM b-mercaptoethanol, and 10³ U/ml ESGRO). The cells were maintained at 37°C in an atmosphere of 5% CO₂ in air.

### Example 2: RT-PCR analysis

Total RNA was isolated using RNeasy Mini Kits (Qiagen) according to the manufacturer's instructions. Synthesis of cDNA was conducted using Omniscript reverse transcriptase (Qiagen) in 20 µl reaction tubes that contained 250 ng total RNA, following the manufacturer's protocol. PCR was performed with Platinum taq polymerase (Invitrogen) according to the manufacturer's protocol. The amplification consisted of 35 cycles at 94°C for 30 seconds, 50°C-60°C for 30 seconds, and 72°C for 30 seconds.

Primer sequences were as follows:

| | |
|---|---|
| Oct3/4 | Forward CTG AGG GCC AGG CAG GAG CAC GAG (SEQ ID NO: 1) |
| | Reverse CTG TAG GGA GGG CTT CGG GCA CTT (SEQ ID NO: 2) |
| Sox2 | Forward GGG TGC AAA AAG AGG AGA GTA G (SEQ ID NO: 3) |
| | Reverse GCG CCT AAC GTA CCA CTA GAA C (SEQ ID NO: 4) |
| c-Myc | Forward CAG AGG AGG AAC GAG CTG AAG CGC (SEQ ID NO: 5) |
| | Reverse TTA TGC ACC AGA GTT TCG AAG CTG TTC (SEQ ID NO: 6) |
| Nanog | Forward AGG GTC TGC TAC TGA GAT GCT CTG (SEQ ID NO:7) |
| | Reverse CAA CCA CTG GTT TTT CTG CCA CCG (SEQ ID NO: 8) |
| Dazl | Forward GCA CTC AGT CTT CAT CAG CAA C (SEQ ID NO: 9) |
| | Reverse CTA TCT TCT GCA CAT CCA CGT C (SEQ ID NO: 10) |
| Stra8 | Forward CCA GTC TGA TAT CAC AGC CTC A (SEQ ID NO: 11) |
| | Reverse TTC CTT GAC CTC CTC TAA GCT G (SEQ ID NO: 12) |
| Tex18 | Forward GGG GAG GGA GTA GTA CCT GTT T (SEQ ID NO: 13) |
| | Reverse CCA CAC CCT GGA TAC TTC ACT T (SEQ ID NO:14) |
| Fig-a | Forward GGA GCG AAG GTC TCA GAG AAA C (SEQ ID NO: 15) |
| | Reverse TGG TAG CTG TAC AAT GGC TCA C (SEQ ID NO: 16) |
| Piwil2 | Forward CCT CCT GTA ACT GGG AAC TTG G (SEQ ID NO: 17) |
| | Reverse GCA CCA CAA CAC CCT ACT ATG A (SEQ ID NO: 18) |
| Rex1 | Forward CAC CAT CCG GGA TGA AAG TGA GAT (SEQ ID NO: 19) |
| | Reverse ACC AGA AAA TGT CGC TTT AGT TTC (SEQ ID NO: 20) |
| Utf1 | Forward CTC AAG GAC AAA CTC CGA GAC T (SEQ ID NO: 21) |
| | Reverse AGA CTT CGT CGT GGA AGA ACT G (SEQ ID NO: 22) |
| c-kit | Forward CAG TGT GTT ACG TGG TCA TGT G (SEQ ID NO: 23) |
| | Reverse GTG TAG CAC TCA AGT CGA AAC G (SEQ ID NO: 24) |
| VASA | Forward CTT GCA GAG ATG TTC AGC AGA C (SEQ ID NO: 25) |
| | Reverse CTC CAA GAG CTT GCT CTC TCT C (SEQ ID NO: 26) |
| Eras | Forward TCT GCG TGA CCA GTG CTT GGC (SEQ ID NO: 27) |
| | Reverse TCT TCA GGC TAC AGA GCA GCC (SEQ ID NO: 28) |
| Cripto | Forward ATG GAC GCA ACT GTG AAC ATG ATG TTC GCA (SEQ ID NO: 29) |
| | Reverse CTT TGA GGT CCT GGT CCA TCA CGT GAC CAT (SEQ ID NO: 30) |
| Esg1 | Forward ATA AGC TTG ATC TCG TCT TCC (SEQ ID NO: 31) |
| | Reverse CTT GCT AGG ATG TAA CAA AGC (SEQ ID NO: 32) |
| FGF4 | Forward CAG CGA GGC GTG GTG AGC ATC TTC GGA (SEQ ID NO: 33) |
| | Reverse CTT CTT GGT CCG CCC GTT CTT ACT GAG (SEQ ID NO: 34) |
| Zfp57 | Forward ATC ACT TGT GCT GCC AAA GAC (SEQ ID NO: 35) |
| | Reverse CTT CTC CTC CTG GAT TCC ATC (SEQ ID NO: 36) |
| PUM1 | Forward TGG AGG CGG CTA TAA TAC TA (SEQ ID NO: 37) |
| | Reverse TTC TTT TCA CCT TTG TCG TT (SEQ ID NO: 38) |
| PUM2 | Forward TAG AAT CTC GGG GAA TGG GA (SEQ ID NO: 39) |
| | Reverse TGG TTT ATT CGC TGT CGG AT (SEQ ID NO: 40) |
| GFR-a | Forward ACT CCT GGA TTT GCT GAT GTC GG (SEQ ID NO: 41) |
| | Reverse CGC TGC GGC ACT CAT CCT T (SEQ ID NO: 42) |
| Zfp145 | Forward TGC TGC CCA ACC ACA AGG (SEQ ID NO: 43) |
| | Reverse CGC TGA ATG AGC CAG TAA AT (SEQ ID NO: 44) |
| b-Actin | Forward CGT GCG TGA CAT CAA AGA GAA GC (SEQ ID NO: 45) |
| | Reverse ATC TGC TGG AAG GTG GAC AGT GAG (SEQ ID NO: 46) |

### Example 3: Immunocytochemistry

The primary antibodies used were: goat anti-GFR"1 (Santa Cruz); rabbit anti-CD9 (Santa Cruz); rabbit anti-VASA (Abcam); and rabbit anti-Notch1 (Santa Cruz). The primary antibodies were used at a 1:50 dilution; Alexa Fluor 594-conjugated secondary antibodies (Invtirogen) were used at a 1:200 dilution. GSCs were cultured on MEFcoated coverslip-slides and fixed with 4% paraformaldehyde for 15 minutes, washed in PBS, and incubated in PBS containing 0.1% Triton X-100 for 10 minutes. To block nonspecific staining, the cells were incubated in 5% FBS prior to being incubated with the primary antibody for 1 hour at room temperature, and then with the secondary antibody for 1 hour at room temperature in the dark. The slides were then coated with mineral oil. Immunoreactivity was detected under a fluorescence microscope.

### Example 4: Flow cytometric analysis

Dissociated GSCs were stained for the presence of surface markers. The primary antibodies used to detect surface markers were: PE-conjugated anti-mouse Thy-1.2 (Becton-Dickinson); PE-conjugated anti-mouse SSEA-1 (Becton-Dickinson); rat antimouse CD9 (Santa Cruz); and rat anti-mouse integrin β1 (Becton-Dickinson).
The primary antibodies were used at a 1:50 dilution. PE-conjugated secondary anti-rat IgG for CD9 and integrin β1 was used at a 1:200 dilution. Briefly, cells were gently resuspended in 0.1 ml of 5% FBS in PBS, incubated with the primary antibody against surface antigens, and later exposed to the appropriate secondary antibody. Isotype-matched FITC/PE tagged controls were also included in each set.
For immunohistochemical analysis of embryoid bodies (EBs), the differentiated EBs were fixed with 4% paraformaldehyde after 10-15 days of plating and stained with 4 antibodies against α-sarcomeric-actinin (1:400, Sigma), Cx43 antibody (Wilgenbus et al., 1992), PECAM (1:800, Pharmingen) and appropriate Cy3- or Cy5-conjugated secondary antibodies (1:400-1:1000, Dianova). Nuclei were stained with Hoechst dye (blue). Samples were imaged using a Zeiss Axiovert 200 microscope equipped with an ApoTome and AxioCam MRm; images were acquired with the Zeiss software AxioVision.

### Example 5: Testicular transplantation

Two to four week-old W/W male mice were used as the recipient mice. Approximately 3×10⁵ cells were injected with a micropipette (80 µm diameter tips) into the seminiferous tubules of the testes of recipient mice through the efferent duct. The mice were sacrificed 3 months later, and the seminiferous tubules were dissociated by collagenase treatment, and examined under a fluorescence microscope to detect Oct4-GFP-positive, transplanted GSCs, demonstrating their colonisation and the restoration of spermatogenesis (cf. also Ogawa et al., Int J Dev Biol 41, 111-112 (1997)).

### Example 6: Karyotyping

Cells were treated with 3 mg/ml Nocodazole for 4 hours, followed by trypsinisation. Single cells were treated with hypotonic (0.56% [w/w]) KCI-solution for 15 minutes, centrifuged, and then fixed with pre-chilled fresh fixative (methanol/acetic acid, 3:1).
The cells were washed three times with fixative and dropped onto glass slides. The slides were air-dried, mounted with VECTASHIELD® Mounting Medium with DAPI (Vector laboratories), and analysed under a fluorescence microscope.

### Example 7: Bisulfite sequencing

One µg of genomic DNA was prepared using the One Day MSP kit (In2Gen) according to the manufacturer's sequencing protocol. PCR amplification was carried out with bisulfite-treated genomic DNAs. The PCR products were subcloned using the PCR 2.1-TOPO vector (Invitrogen). The individual clones were sequenced (GATC biotech).

Primer sequences were as follows:

| | |
|---|---|
| Oct3/4, 1^{st} | Forward GGG ATT TTT AGA TTG GGT TTA GAA AA (SEQ ID NO: 47) |
| | Reverse CCACCCTCTAACCTTAACCTCTAAC (SEQ ID NO: 48) |
| Oct3/4, 2^{nd} | Forward TGAGGAGTGGTTTTAGAAATAATTG (SEQ ID NO: 49) |
| | Reverse AATCCTCTCACCCCTACCTTAAAT (SEQ ID NO: 50) |
| Nanog, 1st | Forward TTT GTA GGT GGG ATT AAT TGT GAA (SEQ ID NO: 51) |
| | Reverse AAA TTT TAA ACA ACA ACC AAA AA (SEQ ID NO: 52) |
| Nanog, 2^{nd} | Forward TTT GTA GGT GGG ATT AAT TGT GAA (SEQ ID NO: 53) |
| | Reverse AAA AAA ACA AAA CAC CAA CCA AAT (SEQ ID NO: 54) |
| Igf2r, 1^{st} | Forward GTA GAG TTT TTT GAA TTT TTT TGT T (SEQ ID NO: 55) |
| | Reverse TAA ACT ATA ATT CTA ATT ATA CCA AAT TAC (SEQ ID NO: 56) |
| Igf2r, 2^{nd} | Forward TGG TAT TTT TAT GTA TAG TTA GGA TAG (SEQ ID NO: 57) |
| | Reverse AAA AAT TCT ATA ATC AAA ACC AAC (SEQ ID NO: 58) |
| H19, 1st | Forward TAA GGA GAT TAT GTT TTA TTT TTG GA (SEQ ID NO: 59) |
| | Reverse CCC CCT AAT AAC ATT TAT AAC CCC (SEQ ID NO: 60) |
| H19, 2^{nd} | Forward AAG GAG ATT ATG TTT TAT TTT TGG A (SEQ ID NO: 61) |
| | Reverse AAA CTT AAA TAA CCC ACA ACA TTA CC (SEQ ID NO: 62) |

### Example 8: In vitro differentiation

Embryoid bodies (EBs) were generated using the hanging drop method (Brustle et al., 1999; Igelmund et al., 1999). The EBs were collected and plated onto 24-well plates (3-4 EBs/well) with 500 µl of DMEM/F12 supplemented with 1 × N2 supplement, 2 mM L-glutamine, 50 µM 2-mercaptoethanol, MEM nonessential amino acid solution, 20 ng/ml mouse EGF, and 10 ng/ml human bFGF. The EBs were maintained at 37°C in an atmosphere of 5% CO₂ in air. For cardiomyocyte differentiation, EBs were either plated on multi-electrode arrays (60 electrodes, 200µm inter electrode distance) or on glass cover slips coated with 0.1% gelatin and cultured for 10-15 days in IMDM+20% FCS. Field potentials from spontaneously beating EBs were recorded with the MEA60 system and the MCRack software (Multichannel Systems) (Igelmund, P. et al., Pflugers Arch 437, 669-79 (1999)). Chronotropic regulation was tested by applying Isoprenaline (ISO, 1-2 µM) and/or 2 µM carbachol (2 µM, CCh). Beating frequencies were calculated with custom written software (Labview 7.1, National Instuments). Action potential recordings were performed with sharp electrodes (50-100MΩ, filled with 3M KCI) impaled into beating areas of EBs and a BA-03X amplifier (NPI electronic). Ca²⁺- transients were recorded from Fura2 AM (2 µM, 30 min) loaded EBs with an imaging system (Till Photonics) as reported earlier (Sasse, P. et al., J Gen Physiol 130, 133-44 (2007)). All physiological recordings were performed at 36±1°C. Recording solution (in mM): NaCl 140, KCI 5.4, CaCl2 2, MgCl2 1, glucose 10, Hepes 10, pH 7.4.

### Example 9: Glial differentiation of gPS cells

Glial Differentiation of gPS cells was performed according to culture conditions established for neural commitment of murine ESC (Brustle, O. et al., Science 285, 754-6 (1999); Glaser, T., Perez-Bouza, A., Klein, K. & Brustle, O., Faseb J 19, 112-4 (2005); Glaser, T., Pollard, S. M., Smith, A. & Brustle, O., PLoS ONE 2, e298 (2007)). Briefly, cells were passaged and propagated on gelatine-coated dishes (0.1%; Sigma) for two days to eliminate MEFs before they were transferred to bacterial culture dishes to allow for EB formation and initiation of differentiation. Four-day-old EBs were plated in defined insulin-transferrin-selenium-fibronectin medium containing 5 µg/ml insulin (Intergene, Purchase, NY), 50 µg/ml human apo-transferrin (Intergene), 30 nM sodium selenite (Sigma) and 2.5 µg/ml fibronectin (Life Technologies, Karlsruhe, Germany). After 4 days, cells were triturated to a single cell suspension and further propagated on polyornithine-coated dishes (15 µg/ml; Sigma) in the presence of 10 ng/ml FGF2, which permits the proliferation of 7 multipotent neural precursors. To obtain glial precursors (GSC-GP), cells were again dissociated and cultured in the presence of 10 ng/ml FGF2 and 20 ng/ml epidermal growth factor (EGF), followed by a 4-day-propagation in the presence of 10 ng/ml FGF2 and 10 ng/ml platelet-derived growth factor (PDGF). Differentiation was induced by a 4- day-growth factor withdrawal in the presence of 3,3,5-triiodothyronine (T3; 30 ng/µl; Sigma) and ascorbic acid (AA; 200 µM; Sigma). All growth factors were purchased from R&D Systems.

### Example 10: Teratoma formation

gPS cells (2 × 106 cells per injection) were subcutaneously injected into 6-week old athymic mice. Four weeks after injection, teratomas were harvested and histologically examined.

### Example 11: Chimera formation

gPS cells were aggregated and cultured with denuded post-compacted 8-cell-stage mouse embryos. Briefly, 8-cell-stage embryos were f!ushed from mice (C57BL/6 × C3H) F1 females × CD1 males] at 2.5 dpc and placed in M2 medium. Clumps of loosely connected ESCs (10-20 cells) from short trypsin-treated Day-2 cultures were selected and transferred into microdrops of KSOM medium with 10% FCS under mineral oil; each clump was placed in a depression in the microdrop. Meanwhile, batches of 30 to 40 embryos were briefly incubated with acidified Tyrode's solution until the zona pellucida had disintegrated. A single embryo was place onto the clump. All aggregates were assembled in this manner, and cultured overnight at 37°C in an atmosphere of 5% CO₂ in air. After 24 hours of culture, the majority of the aggregates had formed blastocysts. Approximately 11 to 14 aggregated embryos were transferred into the uterine horn of each 2.5 dpc pseudopregnant mouse (CD-1 background).

### Example 12: GSC cluster analysis

GSCs cluster analysis was conducted using the previously described methods after slight modification (Yeh, J. R., Zhang, X. & Nagano, M. C., Biol Reprod 77, 897-904 (2007)). For quantification of cluster formation, GSCs were plated in the range of 50-8000 cells in 24-well plate containing MEFs and GSC culture medium. The number of cluster was counted after 7 days of culture. The experiment was 10 performed in three different plates.

### Example 13: Microarray analysis

The microarray study was carried out using Affymetrix Mouse Genome 430 2.0 GeneChip arrays (Affymetrix, Santa Clara, CA) essentially as described before (Ruau, D. et al., Stem Cells (2008)). Briefly, total RNA was extracted from cells with RNAeasy kit including DNase digestion (Qiagen, Hilden, Germany). Biotin-labelled cRNA was obtained from 3 mg of total RNA with the GeneChip One-Cycle labelling kit (Affymetrix). Fifteen micrograms of cRNA were fragmented and hybridized to Affymetrix 430 2.0 GeneChip arrays at 45°C for 16 hrs. DNA chips were washed, stained and scanned using an Affymetrix Fluidics device and GCS3000 scanner, and the images obtained were analyzed using the GCOS software. The experiment was performed in triplicates for the ESCs and iPS cells and in duplicates for the NSCs. Normalization was calculated with RMA algorithm (Irizarry, R. A. et al., Nucleic Acids Res 31, e15 (2003)) implemented in BioConductor.

### Example 14: Endothelial cell differentiation

For endothelial cell differentiation, OG2/LacZ gPS cells were used. Differentiated EBs from gPS cells were digested with collagenase B (Roche Applied Science) to obtain a single cell suspension. CD31 positive cells were isolated by magnetic cell sorting technology according to the manufacturers protocol (Miltenyi Biotec Inc.).

### Material:

### Antibodies for immunocytochemistry, flow cytometric analysis and magnetic cell sorting

The primary antibodies used in this study were: goat anti-GFRa1 (Santa Cruz), rabbit anti-CD9 (Santa Cruz), rabbit anti-mouse VASA (Abcam), rabbit anti-Notch1 (Santa Cruz), PE-conjugated anti-mouse Thy-1.2 (Becton-Dickinson), PE-conjugated anti-mouse SSEA-1 (Becton-Dickinson), rat anti-mouse integrin-β1 (Becton-Dickinson), mouse monoclonal anti-TuJ1 (Chemicon), rabbit anti-Flk1 (Chemicon), mouse monoclonal anti-α-1-fetoprotein (Sigma), anti-α-sarcomeric-actinin (Sigma), rabbit anti-Cx43 antibody (Wilgenbus et al., 1992), anti-PECAM (Pharmingen), mouse anti-04 (Chemicon), rabbit anti-GFAP (DAKO), rabbit anti-mouse proteolipid protein (provided by I.R. Griffiths), rat anti-mouse M2 (Developmental Studies Hybridoma Bank), PE-conjugated anti CD31 antibody (Becton Dickinson), PE-conjugated rat anit-mouse c-kit, rabbit anti-b-galactosidase (Abcam), TRITC-conjugated BS-1 lectin (Sigma), rabbit anti-VE-cadherin (Abcam), rabbit anti-von Willebrand factor (Abcam), and rabbit anti-Nanog (Cosmo Bio Co.). The secondary antibodies were: goat anti-mouse IgM-Cy3 (Jackson Immuno Research), goat anti-rabbit IgG-FITC (Jackson Immuno Research), Alexa Fluor 488-or 594-conjugated secondary antibodies (Molecular Probes), Cy3- or Cy5-conjugated secondary antibodies (Dianova), biotinylated goat anti-rabbit antibody (Dianova), and Alexa 488-Streptavidin (Invitrogen).

### References

1. Takahashi, K. & Yamanaka, S. Induction of pluripotent stem cells from mouse embryonic and adult fibroblast cultures by defined factors. Cell 126, 663-76 (2006).
2. Nakagawa, M. et al. Generation of induced pluripotent stem cells without Myc from mouse and human fibroblasts. Nat Biotechnol 26, 101-6 (2008).
3. Yu, J. et al. Induced pluripotent stem cell lines derived from human somatic cells. Science 318, 1917-20 (2007).
4. Kim, J. et al. Pluripotent stem cells induced from adult neural stem cells by reprogramming with two factors. Nature Accepted (2008).
5. Kanatsu-Shinohara, M. et al. Long-term proliferation in culture and germline transmission of mouse male germline stem cells. Biol Reprod 69, 612-6 (2003).
6. Kubota, H., Avarbock, M. R. & Brinster, R. L. Growth factors essential for selfrenewal and expansion of mouse spermatogonial stem cells. Proc Natl Acad Sci U S A 101, 16489-94 (2004).
7. Kanatsu-Shinohara, M. et al. Generation of pluripotent stem cells from neonatal mouse testis. Cell 119, 1001-12 (2004).
8. Guan, K. et al. Pluripotency of spermatogonial stem cells from adult mouse testis. Nature 440, 1199-203 (2006).
9. Kanatsu-Shinohara, M. et al. Pluripotency of a single spermatogonial stem cell in mice. Biol Reprod 78, 681-7 (2008).
10. Seandel, M. et al. Generation of functional multipotent adult stem cells from GPR125+ germline progenitors. Nature 449, 346-50 (2007).
11. Szabo, P. E., Hubner, K., Scholer, H. & Mann, J. R. Allele-specific expression of imprinted genes in mouse migratory primordial germ cells. Mech Dev 115, 157-60 (2002).
12. Kehler, J. et al. Oct4 is required for primordial germ cell survival. EMBO Rep 5, 1078-83 (2004).
**13**. Yeh, J. R., Zhang, X. & Nagano, M. C. Establishment of a short-term in vitro assay for mouse spermatogonial stem cells. Biol Reprod 77, 897-904 (2007).
14. Brustle, O. et al. Embryonic stem cell-derived glial precursors: a source of myelinating transplants. Science 285, 754-6 (1999).
15. Glaser, T., Perez-Bouza, A., Klein, K. & Brustle, O. Generation of purified oligodendrocyte progenitors from embryonic stem cells. Faseb J 19, 112-4 (2005).
16. Koeppen, A. H., Barron, K. D., Csiza, C. K. & Greenfield, E. A. Comparative immunocytochemistry of Pelizaeus-Merzbacher disease, the jimpy mouse, and the myelin-deficient rat. J Neurol Sci 84, 315-27 (1988).
17. Boison, D. & Stoffel, W. Myelin-deficient rat: a point mutation in exon III (A-C, Thr75----Pro) of the myelin proteolipid protein causes dysmyelination and oligodendrocyte death. Embo J 8, 3295-302 (1989).
18. Duncan, I. D., Grever, W. E. & Zhang, S. C. Repair of myelin disease: strategies and progress in animal models. Mol Med Today 3, 554-61 (1997).
19. Miyamoto, T. et al. Isolation and expression analysis of the testis-specific gene, STRA8, stimulated by retinoic acid gene 8. J Assist Reprod Genet 19, 531-5 (2002).
20. Giuili, G. et al. Murine spermatogonial stem cells: targeted transgene expression and purification in an active state. EMBO Rep 3, 753-9 (2002).
21. Oatley, J. M., Avarbock, M. R., Telaranta, A. I., Fearon, D. T. & Brinster, R. L. Identifying genes important for spermatogonial stem cell self-renewal and survival. Proc Natl Acad Sci U S A 103, 9524-9 (2006).
22. Johnson, J. et al. Oocyte generation in adult mammalian ovaries by putative germ cells in bone marrow and peripheral blood. Cell 122, 303-15 (2005).
23. Igelmund, P. et al. Action potential propagation failures in long-term recordings from embryonic stem cell-derived cardiomyocytes in tissue culture. Pflugers Arch 437, 669-79 (1999).
24. Sasse, P. et al. Intracellular Ca2+ oscillations, a potential pacemaking mechanism in early embryonic heart cells. J Gen Physiol 130, 133-44 (2007).
25. Brustle, O. et al. Embryonic stem cell-derived glial precursors: a source of myelinating transplants. Science 285, 754-6 (1999).
26. Glaser, T., Perez-Bouza, A., Klein, K. & Brustle, O. Generation of purified oligodendrocyte progenitors from embryonic stem cells. Faseb J. 19, 112-4 (2005).
27. Glaser, T., Pollard, S. M., Smith, A. & Brustle, O. Tripotential differentiation of adherently expandable neural stem (NS) cells. PLoS ONE 2, e298 (2007).
28. Yeh, J. R., Zhang, X. & Nagano, M. C. Establishment of a short-term in vitro assay for mouse spermatogonial stem cells. Biol Reprod 77, 897-904 (2007).
29. Ruau, D. et al. Pluripotency associated genes are reactivated by chromatin modifying agents in neurophere cells. Stem Cells (2008).
30. Irizarry, R. A. et al. Summaries of Affymetrix GeneChip probe level data. Nucleic Acids Res 31, e15 (2003).
31. Kanatsu-Shinohara, M., Miki, H., Inoue, K., Ogonuki, N., Toyokuni, S., Ogura, A., and Shinohara, T. (2005). Long-term culture of mouse male germline stem cells under serum-or feeder-free conditions. Biol Reprod 72, 985-991.
32. Wilgenbus, K.K., Kirkpatrick, C.J., Knuechel, R., Willecke, K., and Traub, O. (1992). Expression of Cx26, Cx32 and Cx43 gap junction proteins in normal and neoplastic human tissues. Int J Cancer 51, 522-529.

## Claims

1. A method of generating pluripotent stem cells comprising the steps:
(a) plating germline stem cells (GSCs) at a density of 20,000 cells or less per 1.9 cm²; and
(b) culturing the germline stem cells for at least 11 days without subculturing while maintaining said germline stem cells in an undifferentiated state,
wherein during or after step (b) the presence of markers characteristic of pluripotent stem cells is indicative of the generation of pluripotent stem cells.

2. A method of generating pluripotent stem cells comprising the steps:
(a) plating germline stem cells (GSCs) at a density of 20,000 cells or less per 1.9 cm²; and
(b) culturing the germline stem cells of (a) for 2 to 7 days and subsequently
(ba) transferring and plating said germline stem cells without feeder cells or substitutes thereof; or
(bb) transferring and plating said germline stem cells without feeder cells or substitutes thereof between 2 to 7 days and subsequently transferring and plating said germline stem cells with feeder cells or substitutes thereof;
wherein during or after step (b) the presence of markers characteristic of pluripotent stem cells is indicative of the generation of pluripotent stem cells.

3. The method of claim 1 or 2, comprising an additional step prior to step (a) of culturing GSCs between 2 to 7 days without feeder cells or substitutes thereof at a density of less than 20,000 cells per 1.9 cm².

4. The method of any one of claims 1 to 3, wherein the germline stem cell endogenously expresses Oct3/4 prior to step (a).

5. The method of claim 4, wherein the germline stem cell further endogenously expresses Sox2, KIf4, c-Myc and/or LIN28 prior to step (a).

6. The method of any one of claims 1 to 5, wherein in step (a) the germline stem cells are plated at a density of 500 to 4,000 cells per 1.9 cm².

7. The method of any one of claims 1 to 6, wherein the germline stem cells are obtained by testicular biopsy.

8. The method of any one of claims 1 to 7, wherein the germline stem cells are rodent, bovine, porcine, non-human primate or human germline stem cells.

9. The method of claim 8, wherein the rodent germline stem cells are murine germline stem cells.

10. The method of any one of claims 1 to 9, wherein the cell culture medium used for culturing the GSCs in step (b) comprises the constituents of Tables 1 to 7.

11. A pluripotent stem cell obtainable by the method of any one of claims 1 to 10.

12. A cell obtainable by differentiation of the pluripotent stem cell of claim 11.

13. A method of generating a transgenic non-human animal comprising the steps of:
(a) introducing a pluripotent stem cell according to claim 11 or a pluripotent stem cell generated according to the method of any one of claims 1 to 10 into an non-human preimplantation embryo;
(b) transferring the embryo of step (a) into the uterus of a pseudo-pregnant female non-human animal; and
(c) allowing the embryo to develop and to be born.

14. A transgenic non-human animal generated by the method of claim 13.

15. A composition comprising a pluripotent stem cell according to claim 11 or a pluripotent stem cell obtainable by the method of any one of claims 1 to 10.
